# EUROPEAN PATENT APPLICATION

(11) **EP 3 685 849 A1**
(43) Date of publication of application: **29.07.2020**
(21) Application number: 18859204.2
(22) Date of filing: 20.09.2018
(51) Int. Cl.: A61K 38/29, A61K 9/08, A61K 47/02, A61K 47/12, A61K 47/18, A61K 47/20, A61K 47/22, A61K 47/26, A61K 47/40, A61P 5/18

(54) **TERIPARATIDE-CONTAINING LIQUID PHARMACEUTICAL COMPOSITION HAVING EXCELLENT STABILITY**

(30) Priority: 22.09.2017 JP 2017182611
(71) Applicant: Asahi Kasei Pharma Corporation, Tokyo 100-0006 (JP)
(72) Inventor: MIYABE, Kohei, Tokyo 100-0006 (JP); YAMAGUCHI, Takahiro, Tokyo 100-0006 (JP); MATSUNAWA, Yasuhiro, Tokyo 100-0006 (JP); WATANABE, Narumi, Tokyo 100-0006 (JP)
(74) Representative: Forstmeyer, Dietmar
(86) International application number: PCT/JP2018/034887
(87) International publication number: WO 2019/059302

(57) **Abstract**

According to the present invention, a liquid pharmaceutical preparation containing teriparatide or a salt thereof having excellent physical properties , the liquid pharmaceutical preparation containing teriparatide or a salt thereof, and at least one or more members of inorganic salts and/or organic salts is provided.

## Description

### TECHNICAL FIELD

The present invention relates to a liquid pharmaceutical preparation containing teriparatide or a salt thereof.

### BACKGROUND ART

PTH (parathyroid hormone) is a hormone involved in the regulation of the blood calcium concentration together with calcitonins and vitamin D. As to PTH peptides which are physiologically active equivalents of naturally occurring PTH, PTH peptide-containing freeze-dried preparations and PTH peptide-containing liquid agents have also been known.

### PRIOR ART REFERENCES

### PATENT PUBLICATIONS

Patent Publication 1: Japanese Patent Laid-Open No. Hei-5-306235
Patent Publication 2: Japanese Patent Laid-Open No. 2004-10511
Patent Publication 3: Japanese Patent Laid-Open No. 2007-186466
Patent Publication 4: Japanese Unexamined Patent Publication No. 2001-525372
Patent Publication 5: WO 2012/169435
Patent Publication 6: Japanese Unexamined Patent Publication No. 2012-522059
Patent Publication 7: Japanese Unexamined Patent Publication No. 2015-504087
Patent Publication 8: Japanese Patent Laid-Open No. Sho-63-57527
Patent Publication 9: Japanese Patent Laid-Open No. Hei-2-96533
Patent Publication 10: Japanese Unexamined Patent Publication No. 2004-513069
Patent Publication 11: Japanese Patent Laid-Open No. 2005-213158
Patent Publication 12: WO 2011/139838
Patent Publication 13: Japanese Unexamined Patent Publication No. 2014-507484

### NON-PATENT PUBLICATIONS

Non-Patent Publication 1: Package Insert of Teribone(Registered Trademark) Subcutaneous Injection 56.5 µg (revised November, 2015 (sixth edition, revised on the cautions and the like upon use))
Non-Patent Publication 2: Package Insert of Forteo(Registered Trademark) Subcutaneous Injection Kit 600 µg (revised July, 2014 (seventh edition))
Non-Patent Publication 3: Sung et al., Journal of Biological Chemistry, (1991), 266(5), 2831-2835
Non-Patent Publication 4: Takai et al., Peptide Chemistry 1979, (1980), 187-192
Non-Patent Publication 5: Merrifield, Advances In Enzymology, (1969), 32, 221-296
Non-Patent Publication 6: Robinson et al., Proc. Natl. Acad. Sci. USA, (2001), 98(8), 4367-4372
Non-Patent Publication 7: Vlasak et al., mAbs, (2011), 3(3), 253-263
Non-Patent Publication 8: Tyler-Cross et al., Journal of Biological Chemistry, (1991), 266(33), 22549-22556
Non-Patent Publication 9: Summary of Lectures of Annuαl Meeting of the Society of Nippon Pharmacology, 118th Annual Meeting, 1998, 4, 34
Non-Patent Publication 10: Izutsu et al., Journal of Pharmaceutical Sciences, (2006), 95(4), 781-789
Non-Patent Publication 11: Salgin et al., International Journal of Electrochemical Science, (2012), 7, 12404-12414
Non-Patent Publication 12: Haripada et al., Current Pharmaceutical Biotechnology, (2009), 10, 761-766

### SUMMARY OF THE INVENTION

### PROBLEMS TO BE SOLVED BY THE INVENTION

An object of the present invention is to provide a liquid pharmaceutical preparation containing teriparatide or a salt thereof having excellent physical properties. More specifically, an object of the present invention is to provide 1) the above liquid pharmaceutical preparation in which the formation of a deamidated product of teriparatide or a salt thereof is inhibited; 2) the above liquid pharmaceutical preparation in which the formation of an isomerized product of an aspartic acid residue of teriparatide or a salt thereof is inhibited; 3) the above liquid pharmaceutical preparation which is stable over a long period of time; and 4) the above liquid pharmaceutical preparation in which the formation of an oxidized product of teriparatide or a salt thereof is inhibited. Also, an object of the present invention is to provide 5) teriparatide analogs, a test method in which the presence of the teriparatide analogs and the like is used as an index, and a liquid pharmaceutical preparation containing high-purity teriparatide or a salt thereof, having a reduced content of teriparatide analogs.

### MEANS TO SOLVE THE PROBLEMS

### 1) "Inhibition of Formation of Deamidated Product"

One embodiment of the liquid pharmaceutical preparation of the present invention contains at least one member of inorganic salts and/or organic salts. Further, one embodiment of the liquid pharmaceutical preparation of the present invention has a pH in a given range (for example, from 3.0 to 5.0). In the liquid pharmaceutical preparation as described above, the formation of a deamidated product of teriparatide or a salt thereof can be inhibited.

### 2) "Inhibition of Formation of Isomerized Product of Aspartic Acid Residues"

One embodiment of the liquid pharmaceutical preparation of the present invention contains at least one member of inorganic salts and/or organic salts. Further, one embodiment of the liquid pharmaceutical preparation of the present invention has a pH in a given range (for example, from 3.0 to 5.0). In the liquid pharmaceutical preparation as described above, the formation of an isomerized product of an aspartic acid residue of teriparatide or a salt thereof can be inhibited.

### 3) "Stabilization for Long Period of Time"

One embodiment of the liquid pharmaceutical preparation of the present invention has a pH in a given range (for example, from 4.0 to 5.0). A more specific embodiment is a preparation being filled in a glass container for medical use, not substantially containing a buffer, and having a pH within a given range (for example, from 4.0 to 5.0). Also, an even more specific embodiment is a preparation being filled in a plastic container for medical use, substantially containing a buffer, further containing D-mannitol, and having a pH within a given range (for example, from 4.0 to 5.0). In addition, one embodiment of the liquid pharmaceutical preparation of the present invention contains a specified additive (for example, α-cyclodextrin). The liquid pharmaceutical preparation as described above can be stable for a long period of time.

### 4) "Inhibition of Formation of Oxidized Product"

One embodiment of the liquid pharmaceutical preparation of the present invention contains a given amount of methionine (for example, a mass ratio of teriparatide or a salt thereof to methionine is from 1:0.5 to 1.0). In the liquid pharmaceutical preparation described above, the formation of an oxidized product of teriparatide or a salt thereof (for example, formation of teriparatide oxide in which a methionine residue at the position 8 from an N-terminal of teriparatide or a salt thereof is sulfoxidized) is inhibited. One embodiment of the liquid pharmaceutical preparation of the present invention contains mannitol. In the liquid pharmaceutical preparations described above, the formation of an oxidized product of teriparatide or a salt thereof (for example, formation of an oxidized product of a tryptophan residue at the position 23 in teriparatide or a salt thereof) can be inhibited.

### 5) "Analogs of Teriparatide or Salt Thereof"

One embodiment of the present invention is specified teriparatide analogs (for example, a deamidated product, an Asp isomerized product), a high-quality liquid pharmaceutical preparation in which the analog contents are reduced, and a test method of a liquid pharmaceutical preparation containing teriparatide or a salt thereof, using the presence and/or the existing amounts of the analogs as indices.

Specifically, the present invention relates to the following.
[1] A liquid pharmaceutical preparation containing Component 1 and Component 2:
   wherein Component 1 is teriparatide or salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.
[2] The liquid pharmaceutical preparation according to the above [1], wherein a pH is less than 5.0.
[3] A liquid pharmaceutical preparation containing Component 1 and Component 2, provided that a pH is less than 5.0:
   wherein Component 1 is teriparatide or salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation substantially containing a buffer, provided that when Component 1 is a teriparatide salt, a salt detached from Component 1 does not fall under the buffer.
[4] A liquid pharmaceutical preparation containing Component 1 and Component 2, provided that a pH is less than 5.0:
   wherein Component 1 is teriparatide or salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation not substantially containing a buffer, provided that when Component 1 is a teriparatide salt, a salt detached from Component 1 does not fall under the buffer.
[5] A liquid pharmaceutical preparation containing Component 1 and Component 2, provided that a pH is 5.0 or more:
   wherein Component 1 is teriparatide or salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation not substantially containing a buffer, provided that when Component 1 is a teriparatide salt, a salt detached from Component 1 does not fall under the buffer.
[6] The liquid pharmaceutical preparation according to any one of the above [1] to [5], wherein the mass ratio of Component 1 to Component 2 is 1 : 20 or 20 or more.
[7] A liquid pharmaceutical preparation containing Component 1: wherein Component 1 is teriparatide or a salt thereof, and wherein a pH is from 3.6 to 4.1.
[8] A liquid pharmaceutical preparation containing Component 1 and Component 3:
   wherein Component 1 is teriparatide or a salt thereof; and
   wherein Component 3 is methionine,
   wherein the mass ratio of Component 1 to Component 3 is from 1 : 0.2 to 1.0.
[9] A liquid pharmaceutical preparation containing Component 1 and Component 4:
   wherein Component 1 is teriparatide or a salt thereof; and
   wherein Component 4 is D-mannitol.
[10] A liquid pharmaceutical preparation containing Component 1, wherein Component 1 is teriparatide or a salt thereof, the liquid pharmaceutical preparation being filled in a glass container for medical use, the liquid pharmaceutical preparation not substantially containing a buffer, and having a pH of exceeding 4.0 and 5.0 or less, provided that when Component 1 is a teriparatide salt, a salt detached from Component 1 does not fall under the buffer.
[11] A liquid pharmaceutical preparation containing Component 1: wherein Component 1 is teriparatide or a salt thereof, the liquid pharmaceutical preparation being filled in a plastic container for medical use, and having a pH of from 4.0 to 4.2, the liquid pharmaceutical preparation substantially containing a buffer, provided that when Component 1 is a teriparatide salt, a salt detached from Component 1 does not fall under the buffer, and the liquid pharmaceutical preparation further containing Component 4:
   wherein Component 4 is D-mannitol.
[12] A liquid pharmaceutical preparation containing Component 1 and Component 5:
   wherein Component 1 is teriparatide or a salt thereof; and
   wherein Component 5 is one or more components selected from the group consisting of L-proline, betaine, ectoine, hydroxyectoine, L-arginine hydrochloride, L-histidine, 2-hydroxypropyl-β-cyclodextrin, α-cyclodextrin, β-cyclodextrin, γ-cyclodextrin, N-acetyl-arginine, L-lysine hydrochloride, and N-acetyl-DL-tryptophan.
[13] The liquid pharmaceutical preparation according to the above [12], wherein the Component 5 is one or more components selected from the group consisting of α-cyclodextrin, β-cyclodextrin, N-acetyl-arginine, L-proline, L-arginine hydrochloride, and L-lysine hydrochloride.
[14] The liquid pharmaceutical preparation according to any one of the above [1] to [6], wherein the Component 2 is one or more salts selected from sodium chloride, calcium chloride, magnesium chloride, sodium bromide, sodium acetate, trisodium citrate, and sodium carbonate.
[15] The liquid pharmaceutical preparation according to the above [14], wherein Component 2 is sodium chloride and/or trisodium citrate.
[16] The liquid pharmaceutical preparation according to the above [3] or [4], wherein a pH is 4.1 or more and 4.6 or less.
[17] The liquid pharmaceutical preparation according to the above [5], wherein a pH is 5.0.
[18] The liquid pharmaceutical preparation according to any one of the above [1] to [17], wherein Component 1 is teriparatide acetate.
[19] The liquid pharmaceutical preparation according to any one of the above [1] to [18], wherein the Component 1 content is from 25 to 30 µg, in terms of teriparatide.
[20] The liquid pharmaceutical preparation according to any one of the above [1] to [19], for use in subcutaneous administration in human.
[21] A method for inhibiting a deamidation reaction of Component 1 in a liquid pharmaceutical preparation containing Component 1, including formulating the liquid pharmaceutical preparation to further include Component 2, wherein Component 1 is teriparatide or a salt thereof; and wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.
[22] The method according to the above [21], wherein a pH of the liquid pharmaceutical preparation is less than 5.0.
[23] A method for inhibiting a deamidation reaction of Component 1 in a liquid pharmaceutical preparation containing Component 1 under conditions of a pH of less than 5.0, including formulating the liquid pharmaceutical preparation to further include Component 2, and also substantially include a buffer,
   wherein Component 1 is teriparatide or a salt thereof; and wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under the buffer.
[24] A method for inhibiting a deamidation reaction of Component 1 in a liquid pharmaceutical preparation containing Component 1 under conditions of a pH of less than 5.0, including formulating the liquid pharmaceutical preparation to further include Component 2, but not substantially include a buffer,
   wherein Component 1 is teriparatide or a salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under the buffer.
[25] A method for inhibiting a deamidation reaction of Component 1 in a liquid pharmaceutical preparation containing Component 1 under conditions of a pH of 5.0 or more, including formulating the liquid pharmaceutical preparation to further include Component 2, but not substantially include a buffer,
   wherein Component 1 is teriparatide or a salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under the buffer.
[26] The method according to the above [25], wherein the inhibition of a deamidation reaction is inhibition of formation of a deamidated product of Component 1, wherein the deamidated product has a change of a residue at the position 16 from an N-terminal of Component 1 from an asparagine residue to an isoaspartic acid residue.
[27] The method according to any one of the above [21] to [26], wherein the Component 2 is one or more salts selected from sodium chloride, calcium chloride, magnesium chloride, sodium bromide, sodium acetate, trisodium citrate, and sodium carbonate, provided that Component 2 is a component different from Component 1.
[28] A method for inhibiting a deamidation reaction of Component 1 in a liquid pharmaceutical preparation containing Component 1:
   wherein Component 1 is teriparatide or a salt thereof,
   wherein a pH of the liquid pharmaceutical preparation is adjusted to from 3.6 to 4.1.
[29] A method for inhibiting isomerization of an aspartic acid residue of Component 1 in a liquid pharmaceutical preparation containing Component 1, including formulating the liquid pharmaceutical preparation to further include Component 2,
   wherein Component 1 is teriparatide or a salt thereof; and
   wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.
[30] A method for inhibiting isomerization of an aspartic acid residue of Component 1 in a liquid pharmaceutical preparation containing Component 1, including formulating the liquid pharmaceutical preparation to further include Component 2, and also substantially include a buffer, wherein Component 1 is teriparatide or a salt thereof; and wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under the buffer.
[31] The method according to the above [30], wherein the inhibition of isomerization of an aspartic acid residue is inhibition of isomerization of an aspartic acid residue of Component 1, wherein the isomerization of a residue at the position 30 from an N-terminal of Component 1 is a change from an aspartic acid residue to an isoaspartic acid residue.
[32] The method according to any one of the above [29] to [31], wherein Component 2 is one or more salts selected from sodium chloride, calcium chloride, magnesium chloride, sodium bromide, sodium acetate, trisodium citrate, and sodium carbonate, provided that Component 2 is a component different from Component 1.
[33] A method for inhibiting formation of an oxidized product of Component 1 in a liquid pharmaceutical preparation containing Component 1,
   wherein Component 1 is teriparatide or a salt thereof,
   the method including formulating the liquid pharmaceutical preparation to not substantially include a buffer, provided that when Component 1 is a teriparatide salt, a salt detached from Component 1 does not fall under the buffer, and adjusting a pH of the liquid pharmaceutical preparation to exceeding 4.0 and 5.0 or less, and formulating the liquid pharmaceutical preparation to include Component 3,
   wherein Component 3 is methionine,
   so as to have a mass ratio of Component 1 to Component 3 of from 1 : 0.2 to 1.0, wherein an oxidized product of Component 1 is teriparatide oxidized product in which a methionine residue at the position 8 from an N-terminal of teriparatide is sulfoxidized or a salt thereof.
[34] A method for inhibiting formation of an oxidized product of Component 1 in a liquid pharmaceutical preparation containing Component 1,
   wherein Component 1 is teriparatide or a salt thereof,
   the method including formulating the liquid pharmaceutical preparation to include Component 4,
   wherein Component 4 is D-mannitol,
   wherein the oxidized product of Component 1 is an oxidized product in which a tryptophan residue at the position 23 from an N-terminal of Component 1 is oxidized, and the other residues are identical to the corresponding residues of teriparatide,
[35] A method for storing a liquid pharmaceutical preparation containing Component 1,
   wherein Component 1 is teriparatide or a salt thereof,
   the liquid pharmaceutical preparation being filled in a glass container for medical use, the method including formulating the liquid pharmaceutical preparation to not substantially include a buffer, provided that when Component 1 is a teriparatide salt, a salt detached from Component 1 does not fall under the buffer, and adjusting a pH of the liquid pharmaceutical preparation to exceeding 4.0 and 5.0 or less.
[36] A method for storing a liquid pharmaceutical preparation containing Component 1,
   wherein Component 1 is teriparatide or a salt thereof,
   the liquid pharmaceutical preparation being filled in a plastic container for medical use, the method including formulating the liquid pharmaceutical preparation to substantially include a buffer, provided that when Component 1 is a teriparatide salt, a salt detached from Component 1 does not fall under the buffer, and formulating the liquid pharmaceutical preparation to further include Component 4,
   wherein Component 4 is D-mannitol,
   and adjusting a pH of the liquid pharmaceutical preparation to exceeding 4.0 and 5.0 or less.
[37] A method for storing a liquid pharmaceutical preparation containing Component 1:
   wherein Component 1 is teriparatide or a salt thereof,
   the method including formulating the liquid pharmaceutical preparation to include Component 5:
      wherein Component 5 is one or more components selected from the group consisting of L-proline, betaine, ectoine, hydroxyectoine, L-arginine hydrochloride, L-histidine, 2-hydroxypropyl-β-cyclodextrin, α-cyclodextrin, β-cyclodextrin, γ-cyclodextrin, N-acetyl-arginine, L-lysine hydrochloride, and N-acetyl-DL-tryptophan.
[38] The method according to [37], wherein the Component 5 is one or more components selected from the group consisting of α-cyclodextrin, β-cyclodextrin, N-acetyl-arginine, L-proline, L-arginine hydrochloride, and L-lysine hydrochloride.
[39] An analog of Component 1 wherein a residue at the position 16 from an N-terminal of Component 1 is changed from an asparagine residue to an isoaspartic acid residue, and the other residues are identical to the corresponding residues of teriparatide,
   wherein Component 1 is teriparatide or a salt thereof.
[40] An analog of Component 1 wherein a residue at the position 16 from an N-terminal of Component 1 is changed from an asparagine residue to an aspartic acid residue, and the other residues are identical to the corresponding residues of teriparatide,
   wherein Component 1 is teriparatide or a salt thereof.
[41] An analog of Component 1 wherein a residue at the position 30 from an N-terminal of Component 1 is changed from an aspartic acid residue to an isoaspartic acid residue, and the other residues are identical to the corresponding residues of teriparatide,
   wherein Component 1 is teriparatide or a salt thereof.
[42] An analog of Component 1 wherein a residue at the position 10 from an N-terminal of Component 1 is changed from an asparagine residue to an aspartic acid residue or an isoaspartic acid residue, and a residue at the position 30 from an N-terminal of Component 1 is changed from an aspartic acid residue to an isoaspartic acid residue, and the other residues are identical to the corresponding residues of teriparatide, wherein Component 1 is teriparatide or a salt thereof.
[43] A method for testing quality of a liquid pharmaceutical preparation containing Component 1, including detecting and/or quantifying an analog as defined in any one of the above [39] to [42],
   wherein Component 1 is teriparatide or a salt thereof.
[44] A method for inhibiting formation of an oxidized product in a liquid pharmaceutical syringe preparation containing Component 1, including shading the preparation from light, wherein the oxidized product is a teriparatide oxidized product in which a methionine residue at the position 18 from an N-terminal of teriparatide is sulfoxidized, or a salt thereof,
   wherein Component 1 is teriparatide or a salt thereof.

### ADVANTAGEOUS EFFECTS OF THE INVENTION

According to the present invention, a liquid pharmaceutical preparation containing teriparatide or a salt thereof having excellent physical properties is provided.

### MODES FOR CARRYING OUT THE INVENTION

The present invention shall be described in detail on the basis of specific embodiments. However, the present invention is not intended to be confined to the following embodiments, and can be carried out in any embodiments within the range that would not depart from the spirit of the present invention.

### 1. Liquid Pharmaceutical Preparation:

The present invention provides, as one embodiment, a liquid pharmaceutical preparation containing Component 1, which is teriparatide or a salt thereof; and Component 2, which is at least one or more members of inorganic salts and/or organic salts.

The present invention provides, as one embodiment, a liquid pharmaceutical preparation containing Component 1, which is teriparatide or a salt thereof, and having a pH in a given range (for example, from 3.0 to 5.0).

More preferred specific embodiments of the present invention according to these embodiments will be listed as follows.
1) A liquid pharmaceutical preparation containing Component 1, which is teriparatide or a salt thereof, and Component 2, which is at least one or more members of inorganic salts and/or organic salts, the preparation further substantially containing a buffer, and having a pH of less than 5.0.
2) A liquid pharmaceutical preparation containing Component 1, which is teriparatide or a salt thereof, and Component 2, which is at least one or more members of inorganic salts and/or organic salts, but not substantially containing a buffer, and having a pH of less than 5.0.
3) A liquid pharmaceutical preparation containing Component 1, which is teriparatide or a salt thereof, and Component 2, which is at least one or more members of inorganic salts and/or organic salts, but not substantially containing a buffer, and having a pH of 5.0 or more.
4) The above liquid pharmaceutical preparation, wherein the mass ratio of Component 1 to Component 2 is 1 : 20 or 20 or more.
5) The above liquid pharmaceutical preparation containing Component 1, which is teriparatide or a salt thereof, and having a pH of from 3.6 to 4.1.
6) The above liquid pharmaceutical preparation, wherein the liquid pharmaceutical preparation contains Component 1, which is teriparatide or a salt thereof, has a pH of from 4.0 to 5.0, is filled in a glass container for medical use, but does not substantially contain a buffer.
7) The above liquid pharmaceutical preparation containing Component 1, which is teriparatide or a salt thereof, having a pH of from 4.0 to 5.0, being filled in a plastic container for medical use, and substantially containing a buffer.

The present invention, as one embodiment, contains Component 1, which is teriparatide or a salt thereof, and specified additives.

More preferred specific embodiments of the present invention according to these embodiments will be listed as follows.
1) A liquid pharmaceutical preparation containing Component 1, which is teriparatide or a salt thereof, and Component 3, which is methionine, wherein Component 1 : Component 3 is from 1 : 0.2 to 1.0.
2) A liquid pharmaceutical preparation containing Component 1, which is teriparatide or a salt thereof, and Component 4, which is D-mannitol.
3) A liquid pharmaceutical preparation containing Component 1, which is teriparatide or a salt thereof, and Component 5, which is one or more components selected from the group consisting of L-proline, betaine, ectoine, hydroxyectoine, L-arginine hydrochloride, L-histidine, 2-hydroxypropyl-β-cyclodextrin, α-cyclodextrin, β-cyclodextrin, γ-cyclodextrin, N-acetyl-arginine, L-lysine hydrochloride, and N-acetyl-DL-tryptophan.

The present invention is explained more specifically hereinbelow.

### (1) Liquid Pharmaceutical Preparation:

The liquid pharmaceutical preparation of the present invention is not particularly limited in its form, so long as the liquid pharmaceutical preparation is a liquid pharmaceutical preparation containing teriparatide or a salt thereof (Component 1) described later. Examples include preparations for oral administration (internally ingested agents) or preparations for parenteral administration, and the preparations for parenteral administration are preferred. Examples of the preparations for parenteral administration include preparations for local administration such as external agents, or the preparations for systemic administration such as injections, and the preparations for systemic administration are preferred, and the injections are particularly preferred. In addition, specific routes of administration for systemic administration include intravenous administration, intramuscular administration, intracutaneous administration, subcutaneous administration, and the like, and the subcutaneous administration is preferred. Specifically, examples of the liquid pharmaceutical preparation of the present invention preferably include liquid pharmaceutical preparations for subcutaneous administration or liquid pharmaceutical preparation for injections, and most preferably include liquid pharmaceutical preparations for subcutaneous injections.

In the present invention, the term "pharmaceutical" in a liquid pharmaceutical preparation means a drug used in prevention/treatment/diagnosis of a given disease to a mammal (human, monkey, rat, or the like).

The solvent used in the liquid pharmaceutical preparation of the present invention may be, but not particularly limited to, an aqueous solvent or a non-aqueous solvent, and it is preferred to be an aqueous solvent. In other words, it is preferable that a liquid pharmaceutical preparation of the present invention is an aqueous pharmaceutical preparation. When a liquid pharmaceutical preparation of the present invention is a liquid pharmaceutical preparation for subcutaneous administration, a liquid pharmaceutical preparation for injection, a liquid pharmaceutical preparation for injection for subcutaneous administration, it is particularly preferable that a liquid pharmaceutical preparation of the present invention is an aqueous pharmaceutical preparation, and, for example, the liquid pharmaceutical preparation can be prepared with a water for injection, physiological saline or the like. The aqueous solvent may contain various components such as at least one or more members of inorganic salts and/or organic salts (Component 2), a buffer, and additives (Components 3 to 6), each of which is described later, within the range that would not depart from the spirit of the present invention.

### (2) Teriparatide or Salt Thereof (Component 1):

In the present invention, human PTH(1-34) is a peptide represented by a partial amino acid sequence consisting of amino acid residues of the positions 1 to 34 from the N-terminal side, in the amino acid sequence of human PTH(1-84) which is human parathyroid hormone.

In the present invention, teriparatide means human PTH(1-34) in a free form. Teriparatide can be in a salt form.

In the present invention, the salt of teriparatide includes any salts formed by teriparatide and one or more volatile organic acids. Examples of the volatile organic acid include trifluoroacetic acid, formic acid, acetic acid, and the like. The ratio of teriparatide in a free form to the volatile organic acid is not particularly limited so long as the salt is formed. In particular, as the volatile organic acid, acetic acid is preferred. Specifically, examples of the salt of teriparatide in the present invention include preferably teriparatide acetate.

Since teriparatide or a salt thereof (Component 1) is a peptide, it has an isoelectric point (pI). The measurement of pI can be carried out by a method (for example, a method using HPLC, electrophoresis or the like) that itself is known. In general, the pI of teriparatide or a salt thereof (Component 1) is known to be from 8.3 to 8.4.

The amount of teriparatide or a salt thereof (Component 1) contained in the liquid pharmaceutical preparation of the present invention is not particularly limited, and examples of the amount preferably include as follows. Specifically, the lower limit is preferably 10 µg or more, and more preferably 20 µg or more, 25 µg or more, 27 µg or more, and even more 28 µg or more. In addition, the upper limit is preferably 100 µg or less, and more preferably 50 µg or less, 40 µg or less, 35 µg or less, 30 µg or less, and even more 29 µg or less. Among them, the content of the Component 1 is preferably 28.2 µg or 29.2 µg, in terms of teriparatide. Alternatively, the content of the Component 1 can also be 56.5 µg, in terms of teriparatide. When teriparatide is used as an acetate salt, the amount added with the acetate amount can be exemplified, and in a case of teriparatide pentaacetate, the content of the Component 1 is preferably 30.3 µg or 31.3 µg, in terms of teriparatide acetate. Alternatively, the content of the Component 1 can also be 60.6 µg, in terms of teriparatide acetate.

The content in a unit dose of teriparatide or a salt thereof (Component 1) contained in the liquid pharmaceutical preparation of the present invention is not particularly limited, and examples of the unit dose preferably include as follows. Specifically, the lower limit is more preferably 25 µg or more, 27 µg or more, and even more 28 µg or more. In addition, the upper limit is more preferably 35 µg or less, 30 µg or less, and even more 29 µg or less. In particular, the unit dose of the Component 1 is preferably 28.2 µg, in terms of teriparatide. When teriparatide iswas used as an acetate salt, the amount added with the acetate amount can be exemplified, and in a case of teriparatide pentaacetate, the content in a unit dose of the Component 1 is preferably 30.3 µg, in terms of teriparatide acetate.

The concentration of teriparatide or a salt thereof (Component 1) contained in the liquid pharmaceutical preparation of the present invention is not particularly limited, and examples of the concentration preferably include as follows. Specifically, the lower limit, in terms of teriparatide, is preferably 50 µg/mL or more, more preferably 70 µg/mL or more, 100 µg/mL or more, exceeding 100 µg/mL, 110 µg/mL or more, and even more 120 µg/mL or more. In addition, the upper limit is preferably 500 µg/mL or less, and more preferably 250 µg/mL or less, less than 250 µg/mL, 200 µg/mL or less, 180 µg/mL or less, and even more 160 µg/mL or less. In particular, an example most preferably includes 141 µg/mL.

Teriparatide or a salt thereof (Component 1) contained in the liquid pharmaceutical preparation of the present invention can be produced by methods (for example, methods described in Non-Patent Publications 3 to 5 and the like) that themselves are known.

When "teriparatide or a salt thereof (Component 1)" in the present invention is a teriparatide salt, a salt formed by dissociation of a teriparatide salt in a liquid pharmaceutical preparation of the present invention is not regarded as a buffer in the present invention. Further, the above salt is not regarded as "at least one or more members of inorganic salts and/or organic salts (Component 2)" in the present invention.

### (3) At Least One or More Members of Inorganic Salts and/or Organic Salts (Component 2):

In a liquid pharmaceutical preparation of the present invention, it is preferable to contain at least one or more members of inorganic salts and/or organic salts. In the liquid pharmaceutical preparation as described above, the deamidation of Component 1 and the isomerization of an aspartic acid residue can preferably together be inhibited.

In the present invention, the inorganic salt means a salt formed by binding an inorganic acid to a base. Examples of the inorganic salts include hydrochlorides, hydrobromides, and hydrosulfites, and specific examples include sodium chloride, potassium chloride, ammonium chloride, calcium chloride, magnesium chloride, aluminum chloride, sodium bromide, and sodium hydrosulfite, and most preferably include sodium chloride, calcium chloride, magnesium chloride, and sodium bromide.

In the present invention, the organic salt means a salt formed by binding an organic acid to a base. The organic salts include acetates, citrates, and carbonates. Specific examples include preferably sodium acetate, sodium citrates (monosodium citrate, disodium citrate, and trisodium citrate), and sodium carbonate.

Examples of the inorganic salts and the organic salts in the present invention preferably include sodium salts, potassium salts, ammonium salts, calcium salts, magnesium salts, and aluminum salts.

The organic salts and the inorganic salts in the present invention may be anhydrides, or may be hydrates. For example, magnesium chloride is usually commercially available as a hexahydrate, which can also be directly utilized as an inorganic salt in the present invention, or alternatively, anhydrous magnesium chloride obtained through a dehydration treatment of the hydrate can also be utilized as an inorganic salt.

In the specification of the present application, each of the terms "magnesium chloride hydrate" and "sodium acetate hydrate" means "magnesium chloride hexahydrate" and "sodium acetate trihydrate."

In the present invention, at least one or more members of inorganic salts and/or organic salts mean at least one or more inorganic salts, at least one or more inorganic salts and one or more organic salts, or at least one or more organic salts.

At least one or more members of inorganic salts and/or organic salts include preferably one or more salts selected from sodium salts, calcium salts, and magnesium salts, or one or more salts selected from hydrochlorides, hydrobromides, acetates, citrates, and carbonates, and examples thereof include more preferably sodium salts and/or citrates. Further, specific examples include even more preferably one or more salts selected from sodium chloride, calcium chloride, magnesium chloride, sodium bromide, sodium acetate, trisodium citrate, and sodium carbonate.

For example, Non-Patent Publication 8 discloses that a deamidation reaction of an asparagine residue of a short peptide is not influenced by ionic strength of a liquid reaction mixture (Abstract), and that addition of sodium chloride to the liquid reaction mixture would not inhibit a deamidation reaction (Table III). In addition, a publication referred by Non-Patent Publication 9 has reported that an increase in ionic strength by addition of sodium chloride accelerates the deamidation reaction (McKerrow and Robinson, 1971; Scotchler and Robinson, 1974; page 22553, left column).

On the other hand, in the present invention, the formation of a deamidated product of teriparatide or a salt thereof (Component 1) in the liquid pharmaceutical preparation can be inhibited by addition of one or more members of inorganic salts and/or organic salts to a liquid pharmaceutical preparation. In addition, in the present invention, the isomerized product of an aspartic acid residue of teriparatide or a salt thereof (Component 1) in the liquid pharmaceutical preparation can be inhibited by addition of one or more members of inorganic salts and/or organic salts to a liquid pharmaceutical preparation.

When at least one or more members of inorganic salts and/or organic salts are contained in a liquid pharmaceutical preparation of the present invention, a concentration thereof is not particularly limited. The lower limit is preferably 250 µg/mL or more, 500 µg/mL or more, 1 mg/mL or more, or 2 mg/mL or more, and even more preferably 3 mg/mL or more, and particularly more preferably 5.5 mg/mL or more. On the other hand, the upper limit is preferably 250 mg/mL or less, 100 mg/mL or less, or 25 mg/mL or less, and particularly more preferably 11 mg/mL or less.

When at least one or more members of inorganic salts and/or organic salts (Component 2) are contained in a liquid pharmaceutical preparation of the present invention, a mass ratio of teriparatide or a salt thereof (Component 1) (a mass ratio of Component 1 : Component 2) is not particularly limited. The lower limit is, for example, preferably 1:5 or more, and more preferably 1:10 or more, 1:15 or more, particularly even more preferably 1 : 20 or more, and most preferably 1:35 or more. On the other hand, the upper limit is, for example, preferably 1:500 or less, more preferably 1:300 or less, and most preferably 1:80 or less. When the same salt is contained in a liquid pharmaceutical preparation in a ratio of 1 : 20 or more, the deamidation of Component 1 and the isomerization of an aspartic acid residue of Component 1 can be together remarkably inhibited.

In the present invention, "at least one or more members of inorganic salts and/or organic salts (Component 2)" may be an identical component to a component of a buffer described later, or may be a different component.

### (4) Buffer:

The buffer in the present invention is not particularly limited, so long as the buffer is one that is generally used in the fields of pharmaceuticals, capable of stabilizing a pH of an aqueous solution. The buffer in the present invention includes, for example, acetic acid, tartaric acid, lactic acid, citric acid, boric acid, phosphoric acid, carbonic acid, and salts thereof. More specific examples include acetic acid and sodium acetate, citric acid and trisodium citrate, sodium hydrogencarbonate and sodium carbonate, and sodium dihydrogenphosphate and disodium hydrogenphosphate. The buffer as described above may be contained in a liquid pharmaceutical preparation of the present invention.

In a liquid pharmaceutical preparation of the present invention substantially containing a buffer, the liquid pharmaceutical preparation further containing at least one member of inorganic salts and/or organic salts, the formation of a deamidated product of teriparatide or a salt thereof (Component 1) in the preparation and the formation of an isomerized product of an aspartic acid residue of Component 1 can be together inhibited.

A liquid pharmaceutical preparation of the present invention can be a liquid pharmaceutical preparation not substantially containing a buffer, and in particular a liquid pharmaceutical preparation not substantially containing an acetate buffer is preferred.

The phrase not "substantially" containing a buffer means that the containment of a buffer that cannot inhibit pH fluctuations in the time course caused by, for example, degradation of teriparatide or a salt thereof (Component 1) or other additives, containers filled with components of a drug solution or a reaction product, or a drug solution, or components eluted from the containers to a drug solution, such as an alkali elution when placed in a glass container without measures for alkalis such as a sulfur treatment, and various influences which a drug solution gives to the external environment during storage would not be regarded as containment.

Examples which are regarded as a liquid pharmaceutical preparation not "substantially" containing a buffer include a case of a liquid pharmaceutical preparation containing a buffer in a trace amount. The amount thereof may differ depending on a buffer selected, and it is regarded that a liquid pharmaceutical preparation does not "substantially" contain a buffer even if a liquid pharmaceutical preparation contains a buffer at a concentration of, for example, 1 mM or less, preferably 0.5 mM or less, and even more preferably 0.1 mM or less.

Another example which is regarded as a liquid pharmaceutical preparation not "substantially" containing a buffer includes an embodiment of a liquid pharmaceutical preparation of which pH is within a specified range or a specified value, wherein the preparation contains a buffer at least not having buffering ability in the range of ± 1 each from the lower limit and the upper limit of the specified range or value. For example, it is regarded that a liquid pharmaceutical preparation of the present invention does not "substantially" contain a buffer, in an embodiment of a liquid pharmaceutical preparation of the present invention having a pH of from 4 to 5, when the liquid pharmaceutical preparation contains a buffer that at least does not have buffering ability in the range of a pH of from 3 to 6, or in an embodiment of a liquid pharmaceutical preparation having a pH of 4.6, wherein the liquid pharmaceutical preparation contains a buffer that at least does not have buffering ability in the range of a pH of from 3.6 to 5.6.

Another example which is regarded as a liquid pharmaceutical preparation not "substantially" containing a buffer includes an embodiment of a liquid pharmaceutical preparation of which pH is within a specified range or a specified value, wherein the liquid pharmaceutical preparation contains a buffer so that pKa ± 1 of the buffer is not included or does not overlap in the specified pH range or value. For example, since a pKa of an acetate buffer (actually, pKa of acetic acid) is 4.76, a value of ± 1 thereof would be from 3.76 to 5.76, so that it is regarded that the liquid pharmaceutical preparation would not "substantially" contain an acetate buffer, even when a liquid pharmaceutical preparation having a pH of less than 3.76 or exceeding 5.76 contains an acetate buffer.

Here, since phosphoric acid is a polyvalent acid, and has three kinds of pKa's (2.12, 7.21, and 12.32), the combinations of each of H₃PO₄ and NaH₂PO₄, NaH₂PO₄ and Na₂HPO₄, and Na₂HPO₄ and Na₃PO₄ have buffering actions in individual pH ranges. However, when the combinations are not particularly specified, it is regarded that a liquid pharmaceutical preparation does not "substantially" contain a phosphate buffer even when a liquid pharmaceutical preparation contains a phosphate buffer so that any one of pKa out of the three pKa's has a value of ± 1 is not included or does not overlap in the specified range of pH.

Here, a liquid pharmaceutical preparation of the present invention contains a conjugated base (for example, sodium acetate) of a weak acid (for example, acetic acid), and the conjugated base is not regarded as a buffer in the present invention in a case where the above preparation does not contain the weak acid (for example, acetic acid). However, in a case where the same conjugated base corresponds to "at least one or more members of inorganic salts and/or organic salts (Component 2)" in the present invention mentioned above, the conjugated base is "at least one or more members of inorganic salts and/or organic salts (Component 2)" in the present invention.

### (5) Additives (Components 3 to 6):

A liquid pharmaceutical preparation of the present invention can also be formulated to include various additives. The additives include, for example, solubilizers, stabilizers, isotonic agents, pH adjusting agents, anticorrosives (preservatives), and the like.

Examples of the solubilizers in the present invention include propylene glycol, dipropylene glycol, butylene glycol, polyethylene glycol, polyoxyethylene hydrogenated castor oil, Polyoxyl stearate 40, Povidone, Polysolvate 80, and the like.

Examples of the isotonic agents in the present invention include D-mannitol, sorbitol, glucose, glycerol, propylene glycol, sodium chloride, potassium chloride, and the like.

Examples of the pH adjusting agents in the present invention include diluted hydrochloric acid, sulfuric acid, phosphoric acid, sodium hydroxide, calcium hydroxide, magnesium hydroxide, triethanolamine, and the like.

Example of the anticorrosives (preservatives) in the present invention include methylparaooxybenzoic acid, ethylparaooxybenzoic acid, propylparaooxybenzoic acid, sodium benzoate, phenol, cresol, sorbic acid, benzyl alcohol, and the like.

Examples of the stabilizers in the present invention include mannitol, xylitol, sorbitol, sucrose, glucose, trehalose, maltose, lactose, methionine, histidine, dextran 40, methyl cellulose, gelatin, sodium sulfite, sodium metasulfite, L-proline, betaine, ectoine, hydroxyectoine, L-arginine or hydrochloride thereof, L-histidine or hydrochloride thereof, α-cyclodextrin, β-cyclodextrin, γ-cyclodextrin, 2-hydroxypropyl-β-cyclodextrin, L-lysine or hydrochloride thereof, N-acetyl-DL-tryptophan, and the like. Examples of the stabilizers include preferably L-proline, L-arginine or hydrochloride thereof, α-cyclodextrin, β-cyclodextrin, L-lysine or hydrochloride thereof, mannitol, and methionine, and examples include most preferably α-cyclodextrin, β-cyclodextrin, D-mannitol, and L-methionine.

The content of the stabilizer in the present invention is not particularly limited, and it is preferable that the stabilizer is contained in an amount of from 0.1 to 1000 times or so (mass ratio) of the content of teriparatide or a salt thereof (Component 1) contained in a liquid pharmaceutical preparation of the present invention.

### (5-1) Methionine (Component 3):

A liquid pharmaceutical preparation of the present invention can be formulated to include methionine.

When methionine is added to a liquid pharmaceutical preparation of the present invention, it is preferable that methionine is contained in an amount of 0.1 times (a mass ratio) or more of the content of teriparatide or a salt thereof (Component 1), and more preferably containing in an amount of 0.3 times or more, 0.5 times or more, or 0.7 times or more. The upper limit of the amount of methionine is not particularly limited in the range in which methionine is dissolvable in a liquid pharmaceutical preparation of the present invention, and examples include, for example, preferably 10.0 times or less or 5.0 times or less, even more preferably 2.0 times or less, and most preferably 1.0 time or less. Examples of the amount of methionine to the liquid pharmaceutical preparation of the present invention include preferably from 0.5 to 1.0 time or so (a mass ratio to teriparatide or a salt thereof).

The formation of analogs of teriparatide or a salt thereof (Component 1) (for example, formation of an oxidized product of methionine residue in teriparatide or a salt thereof) can be inhibited if a liquid pharmaceutical preparation of the present invention is formulated to include methionine.

### (5-2) D-Mannitol (Component 4):

A liquid pharmaceutical preparation of the present invention can be formulated to include D-mannitol.

The content of D-mannitol is not particularly limited, and it is preferable that D-mannitol is contained in an amount of from 10 to 1000 times or so (mass ratio) of the content of teriparatide or a salt thereof (Component 1).

The formation of analogs of teriparatide or a salt thereof (Component 1) (for example, formation of an oxidized product of tryptophan residue at the position 23 in teriparatide or a salt thereof) can be inhibited if a liquid pharmaceutical preparation of the present invention is formulated to include D-mannitol.

### (5-3) Specified Additives (Component 5):

A liquid pharmaceutical preparation of the present invention can contain one or more components selected from the group consisting of L-proline, betaine, ectoine, hydroxyectoine, L-arginine or hydrochloride thereof, L-histidine or hydrochloride thereof, 2-hydroxypropyl-β-cyclodextrin, α-cyclodextrin, β-cyclodextrin, γ-cyclodextrin, L-lysine or hydrochloride thereof, N-acetyl-DL-tryptophan, and N-acetyl-arginine (Component 5). The Component 5 is preferably one or more components selected from the group consisting of α-cyclodextrin, β-cyclodextrin, N-acetyl-arginine, L-lysine or hydrochloride thereof, L-proline, and L-arginine or hydrochloride thereof. The liquid pharmaceutical preparation as described above can be stable for a long period of time.

When a liquid pharmaceutical preparation is formulated to include Component 5, it is preferable that the liquid pharmaceutical preparation contains a buffer, and it is preferable to contain, for example, an acetate buffer. Also, when a liquid pharmaceutical preparation is formulated to include these additives, a pH of the liquid pharmaceutical preparation can also take, but not particularly limited to, a preferred range described later.

### (6) pH:

A pH of a liquid pharmaceutical preparation according to the present invention is not particularly limited.

Examples of a pH of the liquid pharmaceutical preparation of the present invention include preferably as follows. Specifically, a pH can be neutral or acidic (a pH of 8.0 or less), and the lower limit is, for example, preferably 3.0 or more, 3.6 or more, 3.8 or more, 4.0 or more, exceeding 4.0, 4.1 or more, 4.2 or more, or 4.4 or more. In addition, the upper limit is, for example, preferably 7.0 or less, less than 7.0, 6.0 or less, 5.0 or less, less than 5.0, 4.9 or less, 4.8 or less, or 4.6 or less. In particular, a pH of 5.0 or less is preferred, and more preferably 3.0 or more and less than 5.0, 3.6 or more and 4.1 or less, 4.0 or more and less than 5.0, or 4.2 or more and less than 5.0, and in particular examples include most preferably within the range of from 4.5 to 4.7.

In a case where PTH or a PTH fragment of teriparatide or the like is used as an aqueous solution agent, the storage stability is worsened, so that conventionally, it is often used as a freeze-dried preparation or as an aqueous solution agent using a specified stabilizer or preservative such as a polyol or m-cresol. In addition, since a peptide or protein is generally instable in an aqueous solution, when it is formed into a liquid pharmaceutical preparation, it is a general practice to add a buffer to a preparation to reduce pH fluctuations (see, Non-Patent Publication 2, Patent Publication 4, and the like).

On the other hand, according to a preferred embodiment of the present invention, in a liquid pharmaceutical preparation containing teriparatide or a salt thereof (Component 1), a liquid pharmaceutical preparation showing stability for a long period of time can be prepared by specifying its pH range to a preferred range defined above, without substantially formulating the above preparation to include a buffer. Here, as mentioned later, if a specified container is used as a container for filling a liquid pharmaceutical preparation of the present invention, it is possible to further improve stability.

A pH of a liquid pharmaceutical preparation of the present invention can be adjusted with, for example, a buffer or a pH adjusting agent by a method that itself is known.

### (7) Method for Producing Liquid Pharmaceutical Preparation:

A liquid pharmaceutical preparation of the present invention can be produced by various methods that themselves are known. Usually, various components mentioned above that constitute a liquid pharmaceutical preparation of the present invention may be appropriately selected, and mixed with a proper solvent to dissolve.

In a case where a liquid pharmaceutical preparation of the present invention is produced as a liquid pharmaceutical preparation for subcutaneous administration, a liquid pharmaceutical preparation for injection, or a liquid pharmaceutical preparation for injection for subcutaneous administration, it is preferable that the liquid pharmaceutical preparation is made into an aqueous liquid pharmaceutical preparation. In the case of an aqueous liquid pharmaceutical preparation, it is preferable that it is subjected to a sterile treatment before administration. As the sterile treatment, when a sterile procedural method is adopted, a liquid pharmaceutical preparation can be produced by dissolving each of weighed raw materials in a water for injection or the like, and subjecting a dissolved solution to a sterile filtration. The water for injection is generally understood as sterile purified water which is compatible to a pyrogen (endotoxin) test, and a water for injection produced by distillation method may be also called as a distilled water for injection in some cases.

This liquid pharmaceutical preparation for injection is further filled and sealed in a washed and sterile treated container, and being subjected to examination, packaging or the like, whereby an injection comprising a liquid pharmaceutical preparation for injection filled therein can be produced. Examples of the container as used herein include, for example, ampules, vials, pre-filled syringes, bags, and the like. The materials of the container include, but not particularly limited to, glass and plastics. Examples of the materials for the container preferably include plastics, from the viewpoint of strength, easiness in handling, safety, and the like.

### (8) Freeze-Drying:

A liquid pharmaceutical preparation of the present invention may embrace embodiments of liquid pharmaceutical preparations reconstituted from freeze-dried preparations. Also, a liquid pharmaceutical preparation of the present invention may not be liquid pharmaceutical preparations which are reconstituted from freeze-dried preparations. Conventionally, it has been known that a freeze-dried preparation containing teriparatide or a salt thereof is dissolved in physiological saline or the like upon use to prepare a liquid pharmaceutical preparation. A liquid pharmaceutical preparation of the present invention may be a redissolved product of a freeze-dried preparation described above (prepared product upon use), or may be a preparation without undergoing a freeze-dried preparation described above (previously liquefied preparation).

### 2. Method for Inhibiting Deamidation of Component 1:

The deamidation means a reaction of non-enzymatically removing an amide in a subject organic compound. A protein or peptide (which may hereinafter be also referred to as a protein or the like) may contain an asparagine residue or a glutamine residue, both of the residues having an amide in a side chain, and a reaction of removing this amide is also included in deamidation (Non-Patent Publication 6). The degradation of a protein or the like in a pharmaceutical preparation of a protein or the like and the consequent reduction in activity thereof are serious matters to be dealt in pharmaceutical industries.

A protein or the like each has different primary to quaternary structures. Moreover, it has been known that the deamidation reaction is dependent on the primary to quaternary structures of subject individual proteins or the like (Non-Patent Publication 6).

Non-Patent Publication 8 discloses that a deamidation reaction of an asparagine residue of a short peptide is not influenced by ionic strength of a liquid reaction mixture (Abstract), and that addition of sodium chloride to the liquid reaction mixture would not inhibit a deamidation reaction (Table III). In addition, a publication referred by Non-Patent Publication 8 has reported that an increase in ionic strength by addition of sodium chloride accelerates the deamidation reaction (McKerrow and Robinson, 1971; Scotchler and Robinson, 1974; page 22553, left column). On the other hand, in the present invention, the deamidation reaction of teriparatide acetate in a liquid pharmaceutical preparation can be remarkably inhibited by adding one or more members of inorganic salts and/or organic salts (Component 2), such as sodium chloride, to a liquid pharmaceutical preparation.

The deamidation of teriparatide or a salt thereof (Component 1) embraces all sorts of biochemical deamidation reactions shown by teriparatide or a salt thereof. The deamidation in the present invention can be a reaction of removing an amide functional group in a side chain of at least one residue out of asparagine residues at the positions 10, 16, and 33 and a glutamine residue at the position 29 from an N-terminal in the amino acid sequence of teriparatide or a salt thereof. In a primary structure of teriparatide, an amino acid residue at a C-terminal side of the asparagine residue is lysine, serine, or phenylalanine.

The deamidation in a side chain of an asparagine residue means a reaction of nitrogen of a peptide bonding formed between the same asparagine residue and an adjoining residue with an amide in a side chain of the same asparagine residue, whereby changing to an aspartic acid residue or an isoaspartic acid residue via a succinimide intermediate. The deamidation in a side chain of a glutamine residue is also the same as a deamidation in a side chain of an asparagine residue.

Examples of the deamidation of Component 1 include preferably, for example, a deamidation reaction in which a residue at the position 16 from an N-terminal of Component 1 is changed from an asparagine residue to an aspartic acid residue, and a deamidation reaction in which a residue at the position 16 from an N-terminal of Component 1 is changed from an asparagine residue to an isoaspartic acid residue.

The inhibition of a deamidation of teriparatide or a salt thereof can be detected and quantified using well-known techniques in accordance with, for example, LC/MS/MS (liquid chromatography/tandem mass spectroscopy).

A preferred mass ratio of Component 1 to Component 2 suitable for inhibiting the deamidation of Component 1 is not particularly limited. As mentioned above, the lower limit is, for example, preferably 1:5 or more, and more preferably 1:10 or more, 1:15 or more, particularly even more preferably 1:20 or more, and most preferably 1:35 or more. On the other hand, the upper limit is, for example, preferably 1:500 or less, more preferably 1:300 or less, and most preferably 1:80 or less. When Component 2 is contained in a liquid pharmaceutical preparation in a ratio of 1:20 or more, the deamidation reaction of Component 1 can be remarkably inhibited.

For the purpose of inhibiting the deamidation of Component 1, Component 2 can be added to a liquid pharmaceutical preparation, and it is preferable to optimize a pH of a liquid pharmaceutical preparation. Examples of a preferred pH for inhibiting the deamidation of Component 1 include the above preferred pH ranges. It is more preferable that a pH is optimized and that a liquid pharmaceutical preparation is added with Component 2.

In order to inhibit the deamidation of Component 1, for example, a pH is preferably adjusted to 4.6 or less, and more preferably adjusted to 4.4 or less, or 4.1 or less. The lower limit is not particularly limited, and, for example, a pH is adjusted to preferably 3.0 or more, and more preferably adjusted to 3.4, or 3.6 or more. For example, when a pH is adjusted to from 3.6 to 4.1, the deamidation can be remarkably suppressed without necessitating that the liquid pharmaceutical preparation is added with Component 2.

For the purpose of inhibiting the deamidation of Component 1, a liquid pharmaceutical preparation may substantially be formulated to include a buffer, or the preparation may not substantially be formulated to include a buffer. However, in a case where a liquid pharmaceutical preparation is added with Component 2, it is preferable that the preparation is formulated to further substantially include a buffer. In particular, when a pH of a liquid pharmaceutical preparation is adjusted to less than 5.0, it is preferable that a liquid pharmaceutical preparation is added with Component 2 and that a liquid pharmaceutical preparation is formulated to substantially include a buffer.

In one embodiment in the present invention, while a liquid pharmaceutical preparation has a pH of less than 5.0 and a liquid pharmaceutical preparation is added with Component 2, the liquid pharmaceutical preparation may be formulated to substantially not include a buffer. In such an embodiment, for example, the specificity of the reaction caused by Component 2 (for example, inhibition of the deamidation reaction of Component 1 or the like, while inhibiting the influences to the isomerization reaction in which a residue at the position 30 from an N-terminal of Component 1 is changed from an aspartic acid residue to an isoaspartic acid residue) can be improved.

In one embodiment in the present invention, while a liquid pharmaceutical preparation has a pH of 5.0 or more and a liquid pharmaceutical preparation is added with Component 2, the liquid pharmaceutical preparation may be formulated to substantially not include a buffer. In such an embodiment, for example, the specificity of the reaction caused by Component 2 can be improved. Here, the specificity of the reaction includes, for example, specificity such as inhibition of the deamidation reaction of Component 1, while inhibiting the influences to the isomerization reaction of Component 1, and the like. Further, remarking on the viewpoint of inhibiting the deamidation reaction of Component 1, examples include preferably the specificity of inhibiting a deamidation reaction in which a residue at the position 16 from an N-terminal of Component 1 is changed from an asparagine residue to isoaspartic acid, while inhibiting the influences to the deamidation reaction in which a residue at the position 16 from an N-terminal of Component 1 is changed from an asparagine residue to aspartic acid.

This method is particularly advantageous when storing a liquid pharmaceutical preparation of the present invention, which can be particularly utilized for the purpose of maintaining the quality of a liquid pharmaceutical preparation when stored at room temperature or under refrigeration over a period of three months or longer, or the like.

### 3. Method for Inhibiting Isomerization of Aspartic Acid Residue of Component 1:

The isomerization of an aspartic acid residue of teriparatide or a salt thereof (Component 1) can be a reaction in which a residue at the position 30 from an N-terminal in the amino acid sequence of teriparatide or a salt thereof is changed from an aspartic acid residue to an isoaspartic acid residue. One of ordinary skill in the art would be able to understand a structural change from an aspartic acid residue to an isoaspartic acid residue (Non-Patent Publication 8 or the like), and the reaction can be detected and quantified using well-known techniques.

A preferred mass ratio of Component 1 to Component 2 for inhibiting the isomerization of an aspartic acid residue of Component 1 (hereinafter appropriately expressed as a ratio of (Component 1) : (Component 2)) is not particularly limited. As mentioned above, the lower limit is, for example, preferably 1:5 or more, and more preferably 1:10 or more, 1:15 or more, particularly even more preferably 1:20 or more, and most preferably 1:35 or more. On the other hand, the upper limit is, for example, preferably 1:500 or less, more preferably 1:300 or less, and most preferably 1:80 or less. When Component 2 is contained in a liquid pharmaceutical preparation in a ratio of 1:20 or more, the isomerization reaction of an aspartic acid residue of Component 1 can be remarkably inhibited.

For the purpose of inhibiting the isomerization of an aspartic acid residue of Component 1, a liquid pharmaceutical preparation can be added with Component 2, and it is preferable that a pH of a liquid pharmaceutical preparation is optimized. Examples of a preferred pH for inhibiting the isomerization of an aspartic acid residue of Component 1 include the above preferred pH ranges. It is more preferable that a liquid pharmaceutical preparation is added with Component 2, and also a pH is optimized.

In order to inhibit the isomerization of an aspartic acid residue of Component 1, for example, a pH is preferably adjusted to 4.6 or less, and more preferably adjusted to 4.4 or less, or 4.1 or less. The lower limit is not particularly limited, and, for example, a pH is adjusted to preferably 3.0 or more, and more preferably adjusted to 3.4, or 3.6 or more. For example, when a pH is adjusted to from 3.6 to 4.1, the isomerization of an aspartic acid residue can be remarkably suppressed without necessitating to add Component 2.

For the purpose of inhibiting the isomerization of an aspartic acid residue of Component 1, a liquid pharmaceutical preparation may be formulated to substantially include a buffer, or a liquid pharmaceutical preparation may be formulated to not include a buffer. However, in a case where a liquid pharmaceutical preparation is added with Component 2, it is preferable that the preparation is formulated to further substantially include a buffer. In particular, when a pH of a liquid pharmaceutical preparation is adjusted to less than 5.0, it is preferable that a liquid pharmaceutical preparation is added with Component 2, and also a liquid pharmaceutical preparation is formulated to further substantially include a buffer. Particularly, in a case of purposing the inhibition of an isomerization reaction in which a residue at the position 30 from an N-terminal of Component 1 is changed from an aspartic acid residue to an isoaspartic acid residue, when a pH is adjusted to less than 5.0, it is very preferable that a liquid pharmaceutical preparation is added with Component 2, and also a liquid pharmaceutical preparation is formulated to further substantially include a buffer.

This method is particularly advantageous when storing a liquid pharmaceutical preparation of the present invention, which can be utilized for the purpose of maintaining the quality of a liquid pharmaceutical preparation when stored at room temperature or under refrigeration particularly over a period of three months or longer.

### 4. Method for Storage:

In a case where PTH is in the form of an aqueous solution agent, the storage stability thereof is worsened, and it is imperative to avoid this disadvantage (Patent Publication 2). In addition, the degradation of a protein or the like in a pharmaceutical preparation of a protein or the like and a consequent reduction in activity can give serious disadvantages in pharmaceutical industries.

For the purposes of increasing the storage stability of a liquid pharmaceutical preparation of the present invention, it is desired to inhibit various analogs of Component 1 which are generated or increased in the process of storing a liquid pharmaceutical preparation. For example, it is preferable that during the storage of a liquid pharmaceutical preparation of the present invention, it is preferable to inhibit the generation or increase of analogs of Component 1, such as a deamidated product of Component 1, a cleaved product of Component 1, an isomerized product of an aspartic acid residue of Component 1, or an oxidized product of Component 1. Therefore, for the purpose of increasing the storage stability of a liquid pharmaceutical preparation of the present invention, it is preferable to take a means of adding at least one component of Components 2 to 5, optimizing a pH, controlling a storage temperature/humidity, or the like.

In particular, for the purpose of increasing the storage stability of a liquid pharmaceutical preparation of the present invention, it is preferable to optimize its pH. For example, a pH of the liquid pharmaceutical preparation can be neutral or acidic (a pH of 8.0 or less), and the lower limit is, for example, preferably 3.0 or more, 3.6 or more, 3.8 or more, 4.0 or more, exceeding 4.0, 4.1 or more, 4.2 or more, or 4.4 or more. In addition, the upper limit is, preferably for example, 7.0 or less, less than 7.0, 6.0 or less, 5.0 or less, less than 5.0, 4.9 or less, 4.8 or less, or 4.6 or less. A preferred range can be, for example, 3.0 or more and 5.0 or less, or 4.0 or more and 5.0 or less.

The container for housing a liquid pharmaceutical preparation when storing is not particularly limited, and it is preferable to be filled in glass medical containers (glass ampules, glass vials, glass syringes, and the like), and plastic medical containers (plastic ampules, plastic vials, plastic syringes, and the like) to be stored.

For the purpose of increasing the storage stability of a liquid pharmaceutical preparation of the present invention, a liquid pharmaceutical preparation may be formulated to substantially include a buffer, or a liquid pharmaceutical preparation may be formulated to not include a buffer. In a case where a liquid pharmaceutical preparation of the present invention is filled in a glass medical container, it is preferable that a liquid pharmaceutical preparation may be formulated to not substantially include a buffer. On the other hand, in a case where a liquid pharmaceutical preparation of the present invention is filled in a plastic medical container, it is preferable that a liquid pharmaceutical preparation is formulated to substantially include a buffer. In these embodiments of the method for storage, it is preferable that a pH at the beginning of storage of a liquid pharmaceutical preparation of the present invention is adjusted to 4.0 or more and 5.0 or less.

For the purpose of increasing the storage stability of a liquid pharmaceutical preparation of the present invention, it is preferable that at least one component of Components 2 to 5 is added to the preparation, and in particular, it is preferable that a liquid pharmaceutical preparation of the present invention is formulated to include at least Component 5.

In a case where a liquid pharmaceutical preparation of the present invention is formulated to include Component 5, a pH at the beginning of storage of a liquid pharmaceutical preparation of the present invention is, but not particularly limited to, for example, preferably 4.0 or more and 5.2 or less. In a case where a liquid pharmaceutical preparation of the present invention is formulated to include Component 5, it is preferable that a liquid pharmaceutical preparation of the present invention is formulated to substantially include a buffer, and more preferably to substantially include an acetate buffer.

In a case where a liquid pharmaceutical preparation of the present invention is formulated to include Component 5, the amount of Component 5 is, but not particularly limited to, preferably from 1:1 to 2000 in a mass ratio of Component 1 : Component 5.

An index for the storage stability of a liquid pharmaceutical preparation of the present invention includes a Component 1 content and a total amount of Component 1 analogs, and the Component 1 content after storage of a certain period (which may be, for example, a relative amount to the Component 1 content at the beginning of storage) or a total amount of Component 1 analogs may be measured/quantified, or the like, whereby the storage stability of a liquid pharmaceutical preparation of the present invention can be evaluated. The total amount of analogs of teriparatide or a salt thereof (Component 1) or the Component 1 amount in a liquid pharmaceutical preparation of the present invention can be detected and quantified using well-known techniques in accordance with, for example, LC/MS/MS (liquid chromatography/tandem mass spectroscopy).

### 5. Method for Inhibiting Formation of Oxidized Product of Component 1:

It has been known that when an aqueous teriparatide solution is added with methionine, teriparatide is stabilized, and that an aqueous teriparatide solution filled in a prefilled syringe made of radiosterilized resin is stabilized with methionine contained in the same solution (Patent Publications 2 and 3).

In a method for inhibiting the formation of an oxidized product of teriparatide or a salt thereof (Component 1) in a liquid pharmaceutical preparation of the present invention, the oxidized product of Component 1 means an oxidized product of at least one or more members of various kinds of oxidized products of Component 1, and examples include, for example, preferably an 8-oxidized product, an 18-oxidized product, an 8-18-oxidized product, and a 23-oxidized product.

The term a 8-oxidized product as used herein means an oxidized product in which a methionine residue at the position 8 from an N-terminal of teriparatide or a salt thereof (Component 1) is sulfoxidized, the term a 8-oxidized product as used herein means an oxidized product in which a methionine residue at the position 18 from an N-terminal of Component 1 is sulfoxidized, and the term a 8-18-oxidized product as used herein means an oxidized product in which methionine residues at the position 8 and the position 18 from an N-terminal of Component 1 are both sulfoxidized, respectively.

The term a 23-oxidized product as used herein means an oxidized product in which a tryptophan residue at the position 23 from an N-terminal of teriparatide or a salt thereof (Component 1) is oxidized.

More specific examples for a 23-oxidized product preferably include any one of analogs 9' to 11' described in Patent Publication 5. Here, the analog 9' described in Patent Publication 5 means a teriparatide analog in which a residue corresponding to tryptophan at the position 23 of teriparatide is the following residue (a), and the other structures are identical to the original teriparatide (human PTH(1-34)-Trp23 [dioxide]). The analog 10' means a teriparatide analog in which a residue corresponding to tryptophan at the position 23 of teriparatide is a residue represented by the following (c)-1 or the following (c)-2, and the other structures are identical to the original teriparatide (human PTH(1-34)-Trp23 [monoxide]). The analog 11' means a teriparatide analog in which a residue corresponding to tryptophan at the position 23 of teriparatide is the following residue (b), and the other structures are identical to the original teriparatide (human PTH(1-34)-Trp23 [dioxide-formate detachment]).

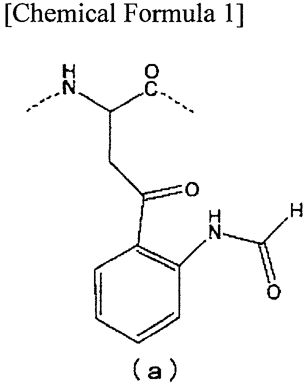

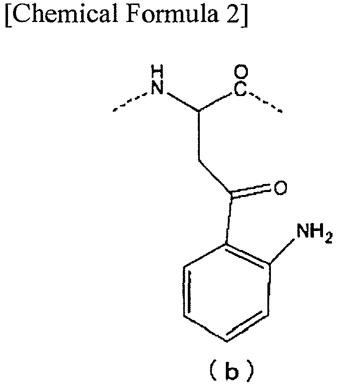

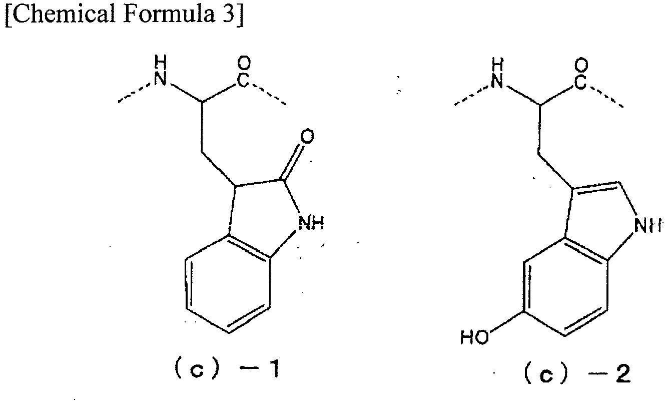

The inhibition of the formation of an oxidized product of Component 1 in a liquid pharmaceutical preparation of the present invention may be, for example, inhibition of the formation of an oxidized product of Component 1 in the preparation during the production of a liquid pharmaceutical preparation of the present invention, or may be the inhibition of the formation an oxidized product of Component 1 in the preparation during transportation / storage of a liquid pharmaceutical preparation of the present invention.

For the purpose of inhibiting the formation of an oxidized product of Component 1 in a liquid pharmaceutical preparation of the present invention, it is preferable that methionine (Component 3) and/or mannitol (Component 4) is added to the preparation.

In a liquid pharmaceutical preparation of the present invention, in a case of purposing the inhibition of the formation of at least one or more oxidized products out of an 8-oxidized product, an 18-oxidized product, and an 8-18-oxidized product, it is preferable that the preparation is formulated to include Component 3. The Component 3 content is not particularly limited, and it is preferable that the mass ratio of Component 1 : Component 3 is 1:0.5 or more. The upper limit of the amount of Component 3 is not particularly limited, and it is preferable that the upper limit is preferably 100 times or less, 50 times or less, 10 times or less, or 5 times or less of Component 1, and preferably within 1 time thereof. Specifically, a liquid pharmaceutical preparation is formulated to include Component 1 and Component 3 so as to have a mass ratio of Component 1 : Component 3 of from 1:0.2 to 1.0.

For the purpose of increasing the inhibition of the formation of an oxidized product of Component 1 in a liquid pharmaceutical preparation of the present invention, it is preferable to optimize its pH. For example, a pH of the liquid pharmaceutical preparation can be neutral or acidic (a pH of 8.0 or less), and the lower limit is, for example, preferably 3.0 or more, 3.6 or more, 3.8 or more, 4.0 or more, exceeding 4.0, 4.1 or more, 4.2 or more, or 4.4 or more. In addition, the upper limit is, for example, preferably 7.0 or less, less than 7.0, 6.0 or less, 5.0 or less, less than 5.0, 4.9 or less, 4.8 or less, or 4.6 or less.

For the purpose of increasing the inhibition of formation of an oxidized product of Component 1 in a liquid pharmaceutical preparation of the present invention, the preparation may be formulated to substantially include a buffer, or a liquid pharmaceutical preparation may be formulated to not include a buffer. For the purpose of increasing the inhibition of formation of an oxidized product of Component 1 in a liquid pharmaceutical preparation of the present invention, it is preferable that a liquid pharmaceutical preparation is formulated to not substantially include a buffer.

In a method for inhibiting the formation of analogs of the present invention, the amount of an oxidized product of teriparatide or a salt thereof (Component 1) can be detected and quantified using well-known techniques in accordance with, for example, LC/MS/MS (liquid chromatography/tandem mass spectroscopy).

This method is particularly advantageous when storing a liquid pharmaceutical preparation of the present invention, which can be particularly utilized for the purpose of maintaining the quality of a liquid pharmaceutical preparation when stored at room temperature or under refrigeration over a period of three months or longer.

### 6. Analogs of Component 1, Test Method Using Analogs as Indices, Preparation in Which Amount of Analog Is Reduced:

In a case where PTH is in the form of an aqueous solution agent, the storage stability is worsened, and it is imperative to avoid this disadvantage (Patent Publication 2). Therefore, providing a new method for controlling the quality of a liquid pharmaceutical preparation containing teriparatide or a salt thereof (Component 1) is useful.

In one embodiment of the present invention, the following analogs (1) to (4), and further a method for testing a liquid pharmaceutical preparation containing Component 1, including the step of detecting or quantifying the presence of at least one of those analogs (for example, a method for testing quality) are provided.

### (1) Deamidated Product (N16→iso-D16):

The deamidated product (N16→iso-D16) means a deamidated product in which a residue at the position 16 from an N-terminal of teriparatide is changed from an asparagine residue to an isoaspartic acid residue, and the other residues are identical to the corresponding residues of teriparatide.

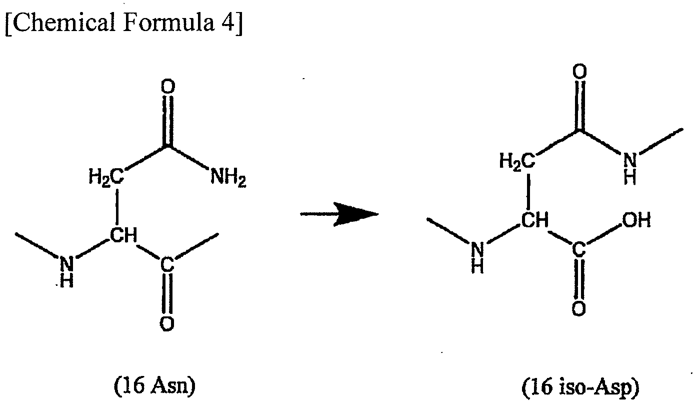

### (2) Deamidated Product (N16→D16):

The deamidated product (N16→D16) means a deamidated product in which a residue at the position 16 from an N-terminal of teriparatide is changed from an asparagine residue to an aspartic acid residue, and the other residues are identical to the corresponding residues of teriparatide.

### (3) Asp Isomerized Product (D30→iso-D30):

Asp isomerized product (D30→iso-D30) means an isomerized product in which a residue at the position 30 from an N-terminal of teriparatide is changed from an aspartic acid residue to an isoaspartic acid residue, and the other residues are identical to the corresponding residues of teriparatide.

### (4) Deamidated Product (N10)-Asp Isomerized Product (D30→ iso-D30):

The deamidated product (N10)-Asp isomerized product (D30→iso-D30) means a deamidated product in which an asparagine residue at the position 10 from an N-terminal of teriparatide is deamidated, whereby consequently the deamidated residue is changed to an aspartic acid residue or an isoaspartic acid residue, and an isomerized product in which a residue at the position 30 from an N-terminal of teriparatide is changed from an aspartic acid residue to an isoaspartic acid residue.

The analogs can be detected and quantified using well-known techniques in accordance with, for example, LC/MS/MS (liquid chromatography/tandem mass spectroscopy). The present method and the present analogs are useful for, for example, controlling the quality of a liquid pharmaceutical preparation containing Component 1.

One embodiment of the present invention provides a liquid pharmaceutical preparation containing Component 1 wherein at least one of the analogs of the above analogs (1) to (4) is reduced. The content of each analog is not particularly limited, and the content is preferably 5% or less, 4% or less, 3% or less, or 2% or less, and more preferably 1% or less, when a total mass of teriparatide and analogs thereof is 100, in terms of percentage (%).

### 7. Photostabilization Method:

There exist at least several thousands or so items of medicaments for medical use that require light-shielding storage. It is preferable that the medicament is stored under sufficient photostabilizing conditions for an appropriate control for a pharmaceutical preparation.

In general, a peptide may be degraded or aggregated by exposure to light (Non-Patent Publication 12). In addition, an aqueous PTH solution which is stable against light and heat is disclosed (Patent Publication 2).

By light-shielding a liquid pharmaceutical preparation of the present invention, for example, storing a liquid pharmaceutical preparation in a state of being packaged in a box, the photostability can be improved. Here, the materials, the structures, the shapes of the box used in packaging are not particularly limited, and the box may be an individually packaged box of pharmaceuticals in which single or plural liquid pharmaceutical preparations are collectively packaged, or may be original corrugated boxes for packaging individually packaged boxes of single or plural pharmaceuticals.

The pharmaceutical individually packaged boxes can be boxes that can prevent breakdown or the like of pharmaceuticals or admixture of foreign matters or the like, or paper boxes including a package insert. It is preferable to provide a perforation or zipper or the like at an unsealing part. The paper materials of the pharmaceutical individually packaged box may be corrugated box or papers.

As one measure for breakdown or the like of pharmaceuticals or admixture of foreign matters or the like, a liquid pharmaceutical preparation is subjected with a blister packaging or a pillow packaging, and then packaged with individually packaged box for pharmaceuticals. Also, examples of the materials for the blister packaging or pillow packaging include, but not particularly limited to, plastic materials.

The materials, structures, shapes, and the like of the original corrugated boxes can be, but not particularly limited to, for example, A flute (A/F), B flute (B/F), or double flute (AB/F). The surface liner can be, for example, C5, K5, K6, or K7, and the inner core can be S120 or S160. A fortified inner core (P160 or the like) may be used in place of the inner core. The surface liner may be a single sided (a single-sided corrugated box), or a double sided (a double-sided corrugated box). Example of the original corrugated box can be preferably A flute (K6 × S120 × K6).

In the photostabilization method or the storage method in a photo-stable state of the present invention, it is preferable that a liquid pharmaceutical preparation is formulated to include methionine, and it is preferable that a liquid pharmaceutical preparation is formulated to further include sodium chloride or a mannitol. In both the methods, the photostability can be measured using analogs of Component 1 contained after storing the liquid pharmaceutical preparation, particularly an amount of 8-oxidized product and/or 18-oxidized product formed as an index. In other words, the extent of photostabilization can be measured by using inhibition of the formation of these oxidized products as an index. It is preferable that the liquid pharmaceutical preparation is a preparation filled in a vial or prefilled syringe made of plastic or glass.

### EXAMPLES

The present invention will be explained more specifically by means of Examples. However, the present invention is not intended to be bound to the following Examples, and the present invention can be carried out in any embodiments within the scope that does not depart from the spirit of the present invention.

Here, in the following Examples, the terms "formulation" and "formulation preparation" may be expressed as a word corresponding to "a liquid pharmaceutical preparation" of the present invention in some cases.

### Example 1 (Preparation of Liquid Pharmaceutical Preparations): (1) Preparation of Liquid Pharmaceutical Preparations Subjected to Test of Inhibiting Formation of Deamidated Product or Isomerized Product of Aspartic Acid Residue:

### (1-1) Production 1:

Formulations 1 to 11 were prepared in accordance with the following Table 1.

A specific preparation method for each formulation is as follows. First, each additive solution listed in the column of "Additives" in the table was mixed with a water for injection. To a mixed solution was added 1 mL of a teriparatide acetate solution (2820 µg/mL in terms of teriparatide), to prepare about 19 mL of a drug solution a. Further, a pH adjusting agent listed in the column of "pH Adjusting Agent" in the table was added to the drug solution a to adjust its pH to that listed in the column of "pH" in the table, and each formulation of which volume was 20 mL was prepared.

Each formulation was subjected to sterile filtration treatment, and a sterile formulation was then filled in plastic syringes in an amount of 0.2 mL each, to produce plastic syringes filled with each formulation (formulation preparations), to be subjected to a test of inhibiting the formation of deamidated products.

The components of each formulation are as listed in the column of "Final Content" in the table.

### [Table 1-1]

**Table 1**

| Formulation | Additives | pH Adjusting Agent | pH | Final Content |
|---|---|---|---|---|
| Formulation 1 | 100 mg/mL D-Mannitol: 6 mL | Hydrochloric acid | 4.6 | Teriparatide: 141 µg/mL |
| | 2 mg/mL L-Methionine: 1 mL | | | D-Mannitol: 30 mg/mL |
| | 44 mg/mL Sodium chloride: 2.5 mL | | | L-Methionine: 100 µg/mL |
| | | | | Sodium chloride: 5.5 mg/mL |
| | | | | Hydrochloric acid: 0.136 mM |
| Formulation 2 | 100 mg/mL D-Mannitol: 9.1 mL | Sodium hydroxide | 4.1 | Teriparatide: 141 µg/mL |
| | | | | D-Mannitol: 45.5 mg/mL |
| | 44 mg/mL Sodium chloride: 2.5 mL | | | Sodium chloride: 5.5 mg/mL |
| | | | | Sodium acetate hydrate: 166 µg/mL |
| | 3.32 mg/mL Sodium acetate hydrate: 1 mL | | | |
| | | | | Acetic acid: 410 µg/mL |
| | 8.2 mg/mL Acetic acid: 1 mL | | | Sodium hydroxide: 0.54 mM |
| Formulation 3 | 100 mg/mL D-Mannitol: 9.1 mL | Sodium hydroxide | 4.6 | Teriparatide: 141 µg/mL |
| | | | | D-Mannitol: 45.5 mg/mL |
| | 44 mg/mL Sodium chloride: 2.5 mL | | | Sodium chloride: 5.5 mg/mL |
| | | | | Sodium acetate hydrate: 467.5 µg/mL |
| | 9.35 mg/mL Sodium acetate hydrate: 1 mL | | | |
| | | | | Acetic acid: 276.5 µg/mL |
| | 5.53 mg/mL Acetic acid: 1 mL | | | Sodium hydroxide: 0.52 mM |
| Formulation 4 | 100 mg/mL D-Mannitol: 9.1 mL | Sodium hydroxide | 5.0 | Teriparatide: 141 µg/mL |
| | | | | D-Mannitol: 45.5 mg/mL |
| | 44 mg/mL Sodium chloride: 2.5 mL | | | Sodium chloride: 5.5 mg/mL |
| | | | | Sodium acetate hydrate: 730 µg/mL |
| | 14.6 mg/mL Sodium acetate hydrate: 1 mL | | | |
| | | | | Acetic acid: 161 µg/mL |
| | 3.22 mg/mL Acetic acid: 1 mL | | | Sodium hydroxide: 0.4 mM |
| Formulation 5 | 100 mg/mL D-Mannitol: 9.1 mL | Sodium hydroxide | 4.6 | Teriparatide: 141 µg/mL |
| | | | | D-Mannitol: 45.5 mg/mL |
| | 44 mg/mL Sodium chloride: 1.25 mL | | | Sodium chloride: 2.75 mg/mL |
| | | | | Sodium acetate hydrate: 467.5 µg/mL |
| | 9.35 mg/mL Sodium acetate hydrate: 1 mL | | | |
| | | | | Acetic acid: 276.5 µg/mL |
| | 5.53 mg/mL Acetic acid: 1 mL | | | Sodium hydroxide: 0.405 mM |
| Formulation 6 | 100 mg/mL D-Mannitol: 9.1 mL | Sodium hydroxide | 4.6 | Teriparatide: 141 µg/mL |
| | 44 mg/mL Sodium chloride: 5 mL | | | D-Mannitol: 45.5 mg/mL |
| | | | | Sodium chloride: 11 mg/mL |
| | 935 mg/mL Sodium acetate hydrate: 1 mL | | | Sodium acetate hydrate: 467.5 µg/mL |
| | 5.53 mg/mL Acetic acid: 1 mL | | | Acetic acid: 276.5 µg/mL |
| | | | | Sodium hydroxide: 0.75 mM |

### [Table 1-2]

**Table 1 (Continued)**

| Formulation | Additives | pH Adjusting Agent | pH | Final Content |
|---|---|---|---|---|
| Formulation 7 | 100 mg/mL D-Mannitol: 6 mL | Hydrochloric acid | 4.6 | Teriparatide: 141 µg/mL |
| | 2 mg/mL L-Methionine: 1 mL | | | D-Mannitol: 30 mg/mL |
| | | | | L-Methionine: 100 µg/mL |
| | | | | Hydrochloric acid: 0.126 mM |
| Formulation 8 | 100 mg/mL D-Mannitol: 6 mL | Hydrochloric acid | 4.1 | Teriparatide: 141 µg/mL |
| | 2 mg/mL L-Methionine: 1 mL | | | D-Mannitol: 30 mg/mL |
| | 44 mg/mL Sodium chloride: 2.5 mL | | | L-Methionine: 100 µg/mL |
| | | | | Sodium chloride: 5.5 mg/mL |
| | | | | Hydrochloric acid: 0.255 mM |
| Formulation 9 | 100 mg/mL D-Mannitol: 6 mL | Hydrochloric acid | 5.0 | Teriparatide: 141 µg/mL |
| | 2 mg/mL L-Methionine: 1 mL | | | D-Mannitol: 30 mg/mL |
| | 44 mg/mL Sodium chloride: 2.5 mL | | | L-Methionine: 100 µg/mL |
| | | | | Sodium chloride: 5.5 mg/mL |
| | | | | Hydrochloric acid: 0.0795 mM |
| Formulation 10 | 100 mg/mL D-Mannitol: 6 mL | Hydrochloric acid | 4.6 | Teriparatide: 141 µg/mL |
| | 2 mg/mL L-Methionine: 1 mL | | | D-Mannitol: 30 mg/mL |
| | 44 mg/mL Sodium chloride: 125 mL | | | L-Methionine: 100 µg/mL |
| | | | | Sodium chloride: 2.75 mg/mL |
| | | | | Hydrochloric acid: 0.135 mM |
| Formulation 11 | 100 mg/mL D-Mannitol: 6 mL | Hydrochloric acid | 4.6 | Teriparatide: 141 µg/mL |
| | 2 mg/mL L-Methionine: 1 mL | | | D-Mannitol: 30 mg/mL |
| | 44 mg/mL Sodium chloride: 5 mL | | | L-Methionine: 100 µg/mL |
| | | | | Sodium chloride: 11 mg/mL |
| | | | | Hydrochloric acid: 0.1375 mM |

### (1-2) Production 2:

Control Formulations 12 to 17 were prepared in accordance with the following Table 2.

A specific preparation method for each formulation is as follows. First, a dissolved solution containing each additive solution listed in the column of "Additives" in the table was mixed with a dissolved solution of teriparatide acetate (28.2 mg in terms of teriparatide), to prepare a drug solution a having a total volume of 175 mL. Thereafter, a pH adjusting agent listed in the column of "pH Adjusting Agent" in the table was added to the drug solution a to adjust its pH to that listed in the column of "pH" in the table, and each formulation of which total volume was 200 mL was prepared.

Each formulation was subjected to sterile filtration treatment, and a sterile formulation was then filled in plastic syringes in an amount of 0.2 mL each, to produce plastic syringes filled with each formulation (formulation preparations), to be subjected to a test of inhibiting the formation of deamidated products.

The components of each formulation are as listed in the column of "Final Content" in the table.

### [Table 2]

**Table 2**

| Formulation | Additives | pH Adjusting Agent | pH | Final Content |
|---|---|---|---|---|
| 12 | 45.5 mg/mL D-Mannitol | Acetic acid | 3.6 | Teriparatide: 141 µg/mL |
| | 45.53 µg/mL Sodium acetate hydrate | | | D-Mannitol: 45.5 mg/mL |
| | | | | Sodium acetate hydrate: 45.53 µg/mL |
| | 460.3 µg/mL Acetic acid | | | |
| | | | | Acetic acid: 668.9 µg/mL |
| 13 | 45.5 mg/mL D-Mannitol | Acetic acid | 3.8 | Teriparatide: 141 µg/mL |
| | 70.43 µg/mL Sodium acetate hydrate | | | D-Mannitol: 45.5 mg/mL |
| | | | | Sodium acetate hydrate: 70.43 µg/mL |
| | 449.3 µg/mL Acetic acid | | | |
| | | | | Acetic acid: 466.7 µg/mL |
| 14 | 45.5 mg/mL D-Mannitol | Sodium acetate hydrate solution | 4.1 | Teriparatide: 141 µg/mL |
| | 166 µg/mL Sodium acetate hydrate | | | D-Mannitol: 45.5 mg/mL |
| | | | | Sodium acetate hydrate: 176 µg/mL |
| | 410 µg/mL Acetic acid | | | |
| | | | | Acetic acid: 410 mL |
| 15 | 45.5 mg/mL D-Mannitol | Sodium acetate hydrate solution | 4.4 | Teriparatide: 141 µg/mL |
| | 280.3 µg/mL Sodium acetate hydrate | | | D-Mannitol: 45.5 mg/mL |
| | | | | Sodium acetate hydrate: 335.3 µg/mL |
| | 356.7 µg/mL Acetic acid | | | |
| | | | | Acetic acid: 356.7 µg/mL |
| 16 | 45.5 mg/mL D-Mannitol | Sodium acetate hydrate solution | 4.6 | Teriparatide: 141 µg/mL |
| | 445.17 µg/mL Sodium acetate hydrate | | | D-Mannitol: 45.5 mg/mL |
| | | | | Sodium acetate hydrate: 480.17 µg/mL |
| | 283.97 µg/mL Acetic acid | | | |
| | | | | Acetic acid: 283.97 µg/mL |
| 17 | 45.5 mg/mL D-Mannitol | Sodium acetate hydrate solution | 5.0 | Teriparatide: 141 µg/mL |
| | 691 µg/mL Sodium acetate hydrate | | | D-Mannitol: 45.5 mg/mL |
| | | | | Sodium acetate hydrate: 796 µg/mL |
| | 175.47 µg/mL Acetic acid | | | |
| | | | | Acetic acid: 175.47 µg/mL |

### (1-3) Production 3:

Formulations 18 to 29 were prepared in accordance with the following Table 3.

A specific preparation method for each formulation is as follows. First, each additive solution/additive listed in the column of "Additives" in the table was mixed with a water for injection. To a mixed solution was added 1 mL of a teriparatide acetate solution (2820 µg/mL in terms of teriparatide) to prepare about 19 mL of a drug solution a. Further, a pH adjusting agent listed in the column of "pH Adjusting Agent" in the table was added to the drug solution a to adjust its pH to that listed in the column of "pH" in the table, and each formulation of which volume was 20 mL was prepared.

Each formulation was subjected to sterile filtration treatment, and a sterile formulation was then filled in plastic syringes in an amount of 0.2 mL each, to produce plastic syringes filled with each formulation (formulation preparations), to be subjected to a test of inhibiting the formation of deamidated products.

The components of each formulation are as listed in the column of "Final Content" in the table.

### [Table 3-1]

**Table 3**

| | Additives | pH Adjusting Agent | pH | Final Content |
|---|---|---|---|---|
| Formulation 18 | 100 mg/mL D-Mannitol: 6 mL | Hydrochloric acid | 4.6 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Calcium chloride: 5.5 mg/mL |
| | Calcium chloride: 0.11 g | | | Hydrochloric acid: 0.1525 mM |
| Formulation 19 | 100 mg/mL D-Mannitol: 6 mL | Hydrochloric acid | 4.6 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Magnesium chloride hydrate: 5.5 mg/mL |
| | Magnesium chloride hydrate: 0.11 g | | | |
| | | | | Hydrochloric acid: 0.13 mM |
| Formulation 20 | 100 mg/mL D-Mannitol: 6 mL | Hydrochloric acid | 4.6 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Sodium bromide: 5.5 mg/mL |
| | Sodium bromide: 0.11 g | | | Hydrochloric acid: 0.133 mM |
| Formulation 21 | 100 mg/mL D-Mannitol: 6 mL | Hydrochloric acid | 4.6 | Teriparatide: 141 µg/mL |
| | | | | D-Mannitol: 30 mg/mL |
| | 2 mg/mL L-Methionine: 1 mL | Sodium hydroxide | | L-Methionine: 100 µg/mL |
| | | | | Sodium carbonate: 5.5 mg/mL |
| | Sodium carbonate: 0.11 g | | | Hydrochloric acid: 103 mM |
| | | | | Sodium hydroxide: 0.14 mM |
| Formulation 22 | 100 mg/mL D-Mannitol: 6 mL | Hydrochloric acid | 4.6 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Sodium acetate hydrate: 5.5 mg/mL |
| | Sodium acetate hydrate: 0.11 g | | | |
| | | | | Hydrochloric acid: 21 mM |
| Formulation 23 | 100 mg/mL D-Mannitol: 6mL | Hydrochloric acid | 4.6 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Trisodium citrate hydrate: 5.5 mg/mL |
| | Trisodium citrate hydrate: 0.11 g | | | |
| | | | | Hydrochloric acid: 26.95 mM |

### [Table 3-2]

**Table 3 (Continued)**

| | Additives | pH Adjusting Agent | pH | Final Content |
|---|---|---|---|---|
| Formulation 24 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Calcium chloride: 5.5 mg/mL |
| | Calcium chloride: 0.11 g | | | Hydrochloric acid: 0.1145 mM |
| Formulation 25 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Magnesium chloride hydrate: 5.5 mg/mL |
| | Magnesium chloride hydrate: 0.11 g | | | |
| | | | | Hydrochloric acid: 0.074 mM |
| Formulation 26 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Sodium bromide: 5.5 mg/mL |
| | Sodium bromide: 0.11 g | | | Hydrochloric acid: 0.0775 mM |
| Formulation 27 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Sodium carbonate: 5.5 mg/mL |
| | Sodium carbonate: 0.11 g | | | Hydrochloric acid: 103.215 mM |
| Formulation 28 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Sodium acetate hydrate: 5.5 mg/mL |
| | Sodium acetate hydrate: 0.11 g | | | |
| | | | | Hydrochloric acid: 12.5 mM |
| Formulation 29 | 100 mg/mL D-Mannitol: 6 mL | Hydrochloric acid | 5.0 | Teriparatide: 141 µg/mL |
| | | | | D-Mannitol: 30 mg/mL |
| | 2 mg/mL L-Methionine: 1 mL | | | L-Methionine: 100 µg/mL |
| | | | | Trisodium citrate hydrate: 5.5 mg/mL |
| | Trisodium citrate hydrate: 0.11 g | | | |
| | | | | Hydrochloric acid: 20.2 mM |

### (2) Preparation of Liquid Pharmaceutical Preparations Subjected to A Test on Effects of Methionine:

Formulations 30 to 32 were prepared in accordance with the following Table 4.

A specific preparation method for each formulation is as follows. First, each additive listed in the column of "Additives" in the table and teriparatide acetate (141 mg in terms of teriparatide) were mixed with a water for injection. After having confirmed the dissolution, the water for injection was used to prepare a drug solution a having a total amount of 980 g. Subsequently, hydrochloric acid was added to the drug solution a to adjust its pH to that listed in the column of "pH" in the table, and water for injection was used to prepare a formulation having a total amount of 1000 g.

Each formulation was subjected to sterile filtration treatment, and a sterile formulation was then filled in plastic syringes in an amount of 0.2 mL each, to produce plastic syringes filled with each formulation (formulation preparations), to be subjected to a test on effects of methionine.

The components of each formulation are as listed in the column of "Final Content" in the table.

### [Table 4]

**Table 4**

| Formulation | Additives | pH | Final Content |
|---|---|---|---|
| Formulation 30 | L-Methionine: 35 mg | 5.0 | Teriparatide: 141 µg/g |
| | Sodium chloride: 10.8 g | | L-Methionine: 35 µg/g |
| | | | Sodium chloride: 10.8 mg/g |
| | | | Hydrochloric acid: 0.12 µmol/g |
| Formulation 31 | L-Methionine: 100 mg | 5.0 | Teriparatide: 141 µg/g |
| | Sodium chloride: 10.8 g | | L-Methionine: 0.1 mg/g |
| | | | Sodium chloride: 10.8 mg/g |
| | | | Hydrochloric acid: 0.12 µmol/g |
| Formulation 32 | D-Mannitol: 57.0 g | 4.1 | Teriparatide: 141 µg/g |
| | | | D-Mannitol: 57 mg/g |
| | | | Hydrochloric acid: 0.192 µmol/g |

### (3) Preparation of Liquid Pharmaceutical Preparations Subjected to Stability Test Relating to Filled Containers:

Formulations 33 to 40 were prepared in accordance with the following Table 5.

A specific preparation method for each formulation is as follows. First, each additive listed in the column of "Additives" in the table was mixed with a water for injection, to prepare a solution a having a total amount of 3000 mL. To 1600 mL of the solution a was dissolved teriparatide acetate (282 mg in terms of teriparatide) to prepare a drug solution a. Thereafter, a diluted hydrochloric acid was added to the drug solution a to adjust its pH to that listed in the column of "pH" in the table, and the above solution a was then used to prepare a formulation having a total amount of 2000 mL.

Each formulation was subjected to sterile filtration treatment, and a sterile formulation was then filled in 2 mL ampules in an amount of 2 mL each, to produce ampules filled with each formulation (formulation ampule preparations), to be subjected to a stability test relating to filled containers. In addition, each formulation was subjected to sterile filtration treatment, and a sterile formulation was then filled in plastic syringes in an amount of 0.2 mL each, to produce plastic syringes filled with each formulation (formulation syringe preparations), to be subjected to a stability test relating to filled containers.

The components of each formulation are as listed in the column of "Final Content" in the table.

### [Table 5]

**Table 5**

| Formulation | Additives | pH | Final Content |
|---|---|---|---|
| Formulation 33 | Sodium chloride: 25.5 g | 4.6 | Teriparatide: 141 µg/mL |
| | Purified white sugar: 75.0 g | | Sodium chloride: 8.5 mg/mL |
| | | | Purified white sugar: 25 mg/mL |
| | L-Methionine: 300 mg | | L-Methionine: 0.1 mg/mL |
| | | | Hydrochloric acid: 0.1395 mM |
| Formulation 34 | Sodium chloride: 33.0 g | 4.6 | Teriparatide: 141 µg/mL |
| | L-Methionine: 300 mg | | Sodium chloride: 11 mg/mL |
| | | | L-Methionine: 0.1 mg/mL |
| | | | Hydrochloric acid: 0.134 mM |
| Formulation 35 | D-Mannitol: 180.0 g | 4.1 | Teriparatide: 141 µg/mL |
| | | | D-Mannitol: 60 mg/mL |
| | | | Hydrochloric acid: 0.208 mM |
| Formulation 36 | Purified white sugar: 312.0 g | 4.1 | Teriparatide: 141 µg/mL |
| | | | Purified white sugar: 104 mg/mL |
| | L-Methionine: 300 mg | | L-Methionine: 0.1 mg/mL |
| | | | Hydrochloric acid: 0.2135 mM |
| Formulation 37 | D-Mannitol: 90.0 g | 4.6 | Teriparatide: 141 µg/mL |
| | Sodium chloride: 16.5 g | | D-Mannitol: 30 mg/mL |
| | L-Methionine: 105 mg | | Sodium chloride: 5.5 mg/mL |
| | | | L-Methionine: 35 µg/mL |
| | | | Hydrochloric acid: 0.13 mM |
| Formulation 38 | D-Mannitol: 90.0 g | 4.6 | Teriparatide: 141 µg/mL |
| | Sodium chloride: 16.5 g | | D-Mannitol: 30 mg/mL |
| | L-Methionine: 300 mg | | Sodium chloride: 5.5 mg/mL |
| | | | L-Methionine: 0.1 mg/mL |
| | | | Hydrochloric acid: 0.1385 mM |
| Formulation 39 | D-Mannitol: 135.0 g | 4.1 | Teriparatide: 141 µg/mL |
| | Purified white sugar: 78.0 g | | D-Mannitol: 45 mg/mL |
| | | | Purified white sugar: 26 mg/mL |
| | L-Methionine: 105 mg | | L-Methionine: 35 µg/mL |
| | | | Hydrochloric acid: 0.205 mM |
| Formulation 40 | Sodium chloride: 16.5 g | 4.6 | Teriparatide: 141 µg/mL |
| | Purified white sugar: 156.0 g | | Sodium chloride: 5.5 mg/mL |
| | | | Purified white sugar: 52 mg/mL |
| | L-Methionine: 300 mg | | L-Methionine: 0.1 mg/mL |
| | | | Hydrochloric acid: 0.1375 mM |

### (4) Preparation of Liquid Pharmaceutical Preparations Subjected to Stability Test Relating to Control Comparisons:

Formulations 41 and 42 were prepared in accordance with the following Table 6.

A specific preparation method for each formulation is as follows. First, each additive solution/additive listed in the column of "Additives" in the table was mixed with a water for injection. To a mixed solution was added a solution containing teriparatide acetate (2820 µg/mL in terms of teriparatide) in an amount listed in the column of "Amount of Teriparatide" in the table, to prepare about 19 mL of a drug solution a. Since a pH of the drug solution a was a pH as listed in the column of "pH" in the table, the pH adjustment was not carried out with a pH adjusting agent, and the volume was further increased with a drug solution a to prepare 20 mL of each formulation.

Each formulation was subjected to sterile filtration treatment, and a sterile formulation was then filled in plastic syringes in an amount of 0.2 mL each, to produce plastic syringes filled with each formulation (formulation preparations), to be subjected to a stability test relating to control comparisons.

### [Table 6]

**Table 6**

| Formulation | Additives | Amount of Teri-paratide | pH Adjusting Agent | pH | Final Content |
|---|---|---|---|---|---|
| Formulation 41 | DL-Methionine: 0.4 g | 213 µL | None | 4.1 | Teriparatide: 30 µg/mL |
| | 20.7 mg/mL Sodium acetate hydrate: 1 mL | | | | DL-Methionine: 20 mg/mL |
| | | | | | Sodium acetate hydrate: 1.035 mg/mL |
| | 50.9 mg/mL Acetic acid: 1 mL | | | | |
| | | | | | Acetic acid: 2.545 mg/mL |
| Formulation 42 | 100 mg/mL D-Mannitol: 9.08 mL | 1.773 mL | None | 4.1 | Teriparatide: 250 µg/mL |
| | | | | | D-Mannitol: 45.5 mg/mL |
| | 3.32 mg/mL Sodium acetate hydrate: 1 mL | | | | Sodium acetate hydrate: 166 µg/mL |
| | 8.2 mg/mL Acetic acid: 1 mL | | | | Acetic acid: 410 µg/mL |
| | | | | | *m*-Cresol: 3 mg/mL |
| | *m*-Cresol: 58 µL | | | | |

### (5) Preparation of Liquid Pharmaceutical Preparations Subjected to Stability Test Relating to Evaluation of Additives:

Formulations 43 to 59 were prepared in accordance with the following Table 7.

A specific preparation method for each formulation is as follows. First, each additive solution/additive listed in the column of "Additives" in the table was mixed with a water for injection. To a mixed solution was added 1 mL of a teriparatide acetate solution (2820 µg/mL in terms of teriparatide), to prepare about 19 mL of a drug solution a. Further, a pH adjusting agent listed in the column of "pH Adjusting Agent" in the table was added to the drug solution a to adjust its pH to that listed in the column of "pH" in the table, and each formulation of which volume was 20 mL was prepared

Each formulation was subjected to sterile filtration treatment, and a sterile formulation was then filled in glass vials in an amount of 0.4 mL each, to produce formulation preparations filled with each formulation, to be subjected to a stability test relating to the evaluation of additives.

The components of each formulation are as listed in the column of "Final Content" in the table. Here, in a case where it is listed in the column of "pH Adjusting Agent" in the table as "None," the pH adjustment was not carried out with a pH adjusting agent, and a pH adjustment was carried out only by increase in volume to adjust its pH as listed in the column of "pH."

### [Table 7-1]

**Table 7**

| Formulation | Additives | pH Adjusting Agent | pH | Final Content |
|---|---|---|---|---|
| Formulation 43 | 16.59 mg/mL Sodium acetate trihydrate: 200 µL | None | 4.0 | Teriparatide: 141 µg/mL |
| | | | | Sodium acetate trihydrate: 165.9 µg/mL |
| | 41 mg/mL Acetic acid: 200 µL | | | |
| | | | | Acetic acid: 410 µg/mL |
| Formulation 44 | 90.81 mg/mL D-Mannitol: 10 mL | Acetic acid | 4.8 | Teriparatide: 141 µg/mL |
| | | | | D-Mannitol: 45.41 mg/mL |
| | 65.3 mg/mL Sodium acetate trihydrate: 200 µL | | | Sodium acetate trihydrate: 653 µg/mL |
| | 19.2 mg/mL Acetic acid: 200 µL | | | Acetic acid: |
| | | | | 192 µg/mL (+ q. s.) |
| Formulation 45 | 90.81 mg/mL D-Mannitol: 10 mL | Acetic acid | 5.2 | Teriparatide: 141 µg/mL |
| | | | | D-Mannitol: 45.41 mg/mL |
| | 86.0 mg/mL Sodium acetate trihydrate: 200 µL | | | Sodium acetate trihydrate: 860 µg/mL |
| | 10.1 mg/mL Acetic acid: 200 µL | | | Acetic acid: |
| | | | | 101 µg/mL (+ q.s.) |
| Formulation 46 | L-Proline: 0.6 g | Acetic acid | 4.0 | Teriparatide: 141 µg/mL |
| | 16.59 mg/mL Sodium acetate trihydrate: 200 µL | | | L-Proline: 30 mg/mL |
| | | | | Sodium acetate trihydrate: 165.9 µg/mL |
| | 41 mg/mL Acetic acid: 200 µL | | | |
| | | | | Acetic acid: 410.9 µg/mL |
| Formulation 47 | Betaine: 0.6 g | Acetic acid | 4.0 | Teriparatide: 141 µg/mL |
| | 16.59 mg/mL Sodium acetate trihydrate: 200 µL | | | Betaine: 30 mg/mL |
| | | | | Sodium acetate trihydrate: 165.9 µg/mL |
| | 41 mg/mL Acetic acid: 200 µL | | | |
| | | | | Acetic acid: 410.7 µg/mL |
| Formulation 48 | Ectoine: 0.75 g | Acetic acid | 4.0 | Teriparatide: 141 µg/mL |
| | 16.59 mg/mL Sodium acetate trihydrate: 200 µL | | | Ectoine: 37.5 mg/mL |
| | | | | Sodium acetate trihydrate: 165.9 µg/mL |
| | 41 mg/mL Acetic acid: 200 µL | | | |
| | | | | Acetic acid: 412.6 µg/mL |

### [Table 7-2]

**Table 7 (Continued)**

| Formulation | Additives | pH Adjusting Agent | pH | Final Content |
|---|---|---|---|---|
| Formulation 49 | Hydroxyectoine: 0.8 g | Acetic acid | 4.0 | Teriparatide: 141 µg/mL |
| | 16.59 mg/mL Sodium acetate trihydrate: 200 µL | | | Hydroxyectoine: 40 mg/mL |
| | | | | Sodium acetate trihydrate: 165.9 µg/mL |
| | 41 mg/mL Acetic acid: 200 µL | | | |
| | | | | Acetic acid: 411.0 µg/mL |
| Formulation 50 | 50 mg/mL L-Arginine hydrochloride: 8 mL | Acetic acid | 4.0 | Teriparatide: 141 µg/mL |
| | | | | L-Arginine hydrochloride: 20 mg/mL |
| | 16.59 mg/mL Sodium acetate trihydrate: 200 µL | | | |
| | | | | Sodium acetate trihydrate: 165.9 µg/mL |
| | 41 mg/mL Acetic acid: 200 µL | | | |
| | | | | Acetic acid: 410.2 µg/mL |
| Formulation 51 | L-Histidine: 600 mg | Acetic acid | 4.0 | Teriparatide: 141 µg/mL |
| | 16.59 mg/mL Sodium acetate trihydrate: 200 µL | | | L-Histidine: 30 mg/mL |
| | | | | Sodium acetate trihydrate: 165.9 µg/mL |
| | 41 mg/mL Acetic acid: 200 µL | | | |
| | | | | Acetic acid: 464.8 µg/mL |
| Formulation 52 | 2-Hydroxypropyl-β-cyclodextrin: 0.3085 g | None | 4.0 | Teriparatide: 141 µg/mL |
| | 16.59 mg/mL Sodium acetate trihydrate: 200 µL | | | 2-Hydroxypropyl-β-cyclodextrin: 15.43 mg/mL |
| | | | | Sodium acetate trihydrate: 165.9 µg/mL |
| | 41 mg/mL Acetic acid: 200 µL | | | Acetic acid: 410 µg/mL |
| Formulation 53 | γ-Cyclodextrin: 3.0225 g | Acetic acid | 4.0 | Teriparatide: 141 µg/mL |
| | 16.59 mg/mL Sodium acetate trihydrate: 200 µL | | | γ-Cyclodextrin: 151.13 mg/mL |
| | | | | Sodium acetate trihydrate: 165.9 µg/mL |
| | 41 mg/mL Acetic acid: 200 µL | | | |
| | | | | Acetic acid: 410.0 µg/mL |
| Formulation 54 | 4.6 mg/mL α-Cyclodextrin: 5 mL | Acetic acid | 4.0 | Teriparatide: 141 µg/mL |
| | | | | α-Cyclodextrin: 1.15 mg/mL |
| | 16.6 mg/mL Sodium acetate trihydrate: 200 µL | | | Sodium acetate trihydrate: 166 µg/mL |
| | | | | |
| | 41 mg/mL Acetic acid: 200 µL | | | Acetic acid: 410.2 µg/mL |

### [Table 7-3]

**Table 7 (Continued)**

| Formulation | Additives | pH Adjusting Agent | pH | Final Content |
|---|---|---|---|---|
| Formulation 55 | 53 mg/mL β-Cyclodextrin: 5 mL | Acetic acid | 4.0 | Teriparatide: 141 µg/mL |
| | | | | β-Cyclodextrin: 1.3 mg/mL |
| | 16.6 mg/mL Sodium acetate trihydrate: 200 µL | | | Sodium acetate trihydrate: 166 µg/mL |
| | 41 mg/mL Acetic acid: 200 µL | | | Acetic acid: 410.2 µg/mL |
| Formulation 56 | 5 mg/mL L-Lysine hydrochloride: 10 mL | None | 4.0 | Teriparatide: 141 µg/mL |
| | 16.6 mg/mL Sodium acetate trihydrate: 200 µL | | | L-Lysine hydrochloride: 2.5 mg/mL |
| | 41 mg/mL Acetic acid: 200 µL | | | Sodium acetate trihydrate: 166 µg/mL |
| | | | | Acetic acid: 410 µg/mL |
| Formulation 57 | 0.933 mg/mL N-Acetyl-DL-tryptophan: 18 mL | Sodium acetate trihydrate | 4.0 | Teriparatide: 141 µg/mL |
| | 16.6 mg/mL Sodium acetate trihydrate: 200 µL | | | N-Acetyl-DL-tryptophan: 0.84 mg/mL |
| | 41 mg/mL Acetic acid: 200 µL | | | Sodium acetate trihydrate: 566 µg/mL |
| | | | | Acetic acid: 410 µg/mL |
| Formulation 58 | 0.466 mg/mL N-Acetyl-DL-tryptophan: 18 mL | Sodium acetate trihydrate | 5.0 | Teriparatide: 141 µg/mL |
| | 69.09 mg/mL Sodium acetate trihydrate: 200 µL | | | N-Acetyl-DL-tryptophan: 0.42 mg/mL |
| | 17.55 mg/mL Acetic acid: 200 µL | | | Sodium acetate trihydrate: 1340.9 µg/mL |
| | | | | Acetic acid: 175.5 µg/mL |
| Formulation 59 | 0.466 mg/mL N-Acetylarginine: 7.4 mg | Acetic acid | 4.0 | Teriparatide: 141 µg/mL |
| | | | | N-Acetylarginine: 0.37 mg/mL |
| | 16.6 mg/mL Sodium acetate trihydrate: 200 µL | | | Sodium acetate trihydrate: 166 µg/mL |
| | 41 mg/mL Acetic acid: 200 µL | | | Acetic acid: 410 µg/mL |

Further, Formulations 60 and 61 were prepared in accordance with the following Table 8.

A specific preparation method for each formulation is as follows. An amount of teriparatide listed in "Additive" in the table was weighed, and then dissolved with a solvent listed in the column of "Solvent" in the table, and the volume was increased to that listed in the column of "Increased volume to" in the table, to prepare each formulation.

Thereafter, each formulation was subjected to sterile filtration treatment, and a sterile formulation was then filled in glass vials in an amount of 0.4 mL each, to produce formulation preparations filled with each formulation, to be subjected to a stability test relating to the evaluation of additives.

### [Table 8]

**Table 8**

| Formulation | Additives | Solvent | Increased volume to: | Teriparatide Final Content |
|---|---|---|---|---|
| Formulation 60 | Teriparatide: 2.82 mg | Water for injection | 20 mL | 141 µg/mL |
| Formulation 61 | Teriparatide: 5.64 mg | 50% DMSO | 20 mL | 282 µg/mL |

### (6) Preparation of Control Liquid Pharmaceutical Preparation:

Formulation 62 was prepared in accordance with the following Table 9.

A specific preparation method for each formulation is as follows. First, each additive listed in the column of "Additives" in the table was mixed with a water for injection, to prepare a solution a having a total amount of 3000 g. To 2480 g of the solution a was dissolved teriparatide acetate (352.5 mg in terms of teriparatide), and the solution a was used to make a total amount of 2500 g, to prepare Formulation 62.

Formulation 62 was subjected to sterile filtration treatment, and a sterile formulation was then filled in plastic syringes in an amount of 0.2 mL each, and syringes filled with Formulation 62 were utilized as Formulation 62 Preparation.

### [Table 9]

**Table 9**

| Formulation | Additives | pH | Final Content |
|---|---|---|---|
| Formulation 62 | D-Mannitol: 136.5 g | 4.1 | Teriparatide: 141 µg/g |
| | Glacial acetic acid: 1230 mg | | D-Mannitol: 45.5 mg/g |
| | Sodium acetate hydrate: 498 mg | | Glacial acetic acid: 0.41 mg/g |
| | | | Sodium acetate hydrate: 0.166 mg/g |

### Example 2 (Test of Inhibiting Formation of Deamidated Product and Isomerized Product of Aspartic Acid Residue):

### (1) Test Method:

The tests for inhibiting formation of the deamidated product and the isomerized product of aspartic acid residue were carried out using Formulation 1 to 29 Preparations prepared in "Liquid Pharmaceutical Preparations Subjected to Test of Inhibiting Formation of Deamidated Product and Isomerized Product of Aspartic Acid Residue" mentioned above.

Specifically, each formulation preparation was stored in a stability tester at 25°C / 60% RH, and samples were then taken in the time course. The stability was measured by high-performance liquid chromatography, and produced impurities were analyzed.

### (2) Test Results:

The test results are shown in the following Tables 10 to 14. The numerical figure in the table shows a proportion (%) when a total amount of teriparatide and analogs thereof contained in the formulation is defined as 100.

The expression "Deamidated Product (N16 → iso-D16)" in the table means a deamidated product in which an asparagine residue at the position 16 from an N-terminal of teriparatide is deamidated, thereby consequently changing from the asparagine residue to an isoaspartic acid residue.

The expression "Deamidated Product (N16 → D16)" in the table means a deamidated product in which an asparagine residue at the position 16 from an N-terminal of teriparatide is deamidated, thereby consequently changing from the asparagine residue to an aspartic acid residue.

The expression "Asp Isomerized Product (D30 → iso-D30)" in the tables means an isomerized product of an aspartic acid residue in which a residue at the position 30 from an N-terminal of teriparatide is changed from an aspartic acid residue to an isoaspartic acid residue.

In addition, the expression "Deamidated Product (N10)-Asp Isomerized Product (D30 → iso-D30)" in the following list means a deamidated product in which an asparagine residue at the position 10 from an N-terminal of teriparatide is deamidated, thereby consequently changing from the asparagine residue to an aspartic acid residue or an isoaspartic acid residue, and an isomerized product of an aspartic acid residue in which a residue at the position 30 from an N-terminal of teriparatide is changed from an aspartic acid residue to an isoaspartic acid residue.

The expression "Deamidated Product" in the tables means a collective name for Deamidated Product (N10) - Asp Isomerized Product (D30 → iso-D30), Deamidated Product (N16 → iso-D16), and Deamidated Product (N16 → D16) (i.e., the numerical value in the tables for the deamidated product meaning a total amount of the above three(3) compounds). The expression "Asp Isomerized Product" in the tables means a collective name for Deamidated Product (N10) - Asp Isomerized Product (D30 → iso-D30) and Asp Isomerized Product (D30 → iso-D30) (i.e., the numerical value in the tables for the Asp isomerized product meaning a total amount of the above two(2) compounds).

### [Table 10-1]

**Table 10**

| Formulation | Conc. (mg/ml) ofNaCl in Formulation | Buffer, Present or Absent | pH | Deamidated Product | | | Asp Isomerized Product | | |
|---|---|---|---|---|---|---|---|---|---|
| | | | | At Beginning | First mo. | Third mo. | At Beginning | First mo. | Third mo. |
| Formulation 7 | None | Absent | 4.6 | 0.03 | 0.72 | 2.83 | 0.07 | 0.31 | 1.91 |
| Formulation 1 | 5.5 | Absent | 4.6 | 0.03 | 0.41 | 1.6 | 0.08 | 0.27 | 1.67 |
| Formulation 16 | None | Present | 4.6 | 0.09 | 0.92 | 2.85 | 0.08 | 0.55 | 2.12 |
| Formulation 3 | 5.5 | Present | 4.6 | 0.03 | 0.36 | 1.17 | 0.07 | 0.24 | 1.41 |

### [Table 10-2]

**Table 10 (Continued)**

| Formulation | Conc. (mg/ml) ofNaCl in Formulation | Buffer, Present or Absent | pH | Deamidated Product (N16 → iso-D16) | | | Asp Isomerized Product (N16 → D16) | | |
|---|---|---|---|---|---|---|---|---|---|
| | | | | At Beginning | First mo. | Third mo. | At Beginning | First mo. | Third mo. |
| Formulation 7 | None | Absent | 4.6 | 0.03 | 0.56 | 1.68 | 0 | 0.14 | 0.57 |
| Formulation 1 | 5.5 | Absent | 4.6 | 0.03 | 0.3 | 0.92 | 0 | 0.07 | 0.34 |
| Formulation 16 | None | Present | 4.6 | 0.05 | 0.55 | 1.61 | 0 | 0.18 | 0.55 |
| Formulation 3 | 5.5 | Present | 4.6 | 0.03 | 0.25 | 0.67 | 0 | 0.06 | 0.26 |

### [Table 10-3]

**Table 10 (Continued)**

| Formulation | Conc. (mg/ml) of NaCl in Formulation | Buffer, Present or Absent | pH | Asp Isomerized Product (D30 → iso-D30) | | |
|---|---|---|---|---|---|---|
| | | | | At Beginning | First mo. | Third mo. |
| Formulation 7 | None | Absent | 4.6 | 0.07 | 0.29 | 1.33 |
| Formulation 1 | 5.5 | Absent | 4.6 | 0.08 | 0.23 | 1.33 |
| Formulation 16 | None | Present | 4.6 | 0.04 | 0.36 | 1.43 |
| Formulation 3 | 5.5 | Present | 4.6 | 0.07 | 0.19 | 1.17 |

### [Table 11-1]

**Table 11**

| Formulation | Conc. (mg/ml) ofNaCl in Formulation | Buffer, or Absent | pH | Deamidated Product | | | Asp Isomerized Product | | |
|---|---|---|---|---|---|---|---|---|---|
| | | | | At Beginning | First mo. | Third mo. | At Beginning | First mo. | Third mo. |
| Formulation 7 | None | Absent | 4.6 | 0.03 | 0.72 | 2.83 | 0.07 | 0.31 | 1.91 |
| Formulation 10 | 2.75 | Absent | 4.6 | 0.03 | 0.42 | 1.78 | 0.07 | 0.24 | 1.66 |
| Formulation 1 | 5.5 | Absent | 4.6 | 0.03 | 0.41 | 1.6 | 0.08 | 0.27 | 1.67 |
| Formulation 11 | 11 | Absent | 4.6 | 0.03 | 0.34 | 1.37 | 0.07 | 0.23 | 1.6 |

### [Table 11-2]

**Table 11 (Continued)**

| Formulation | Conc. (mg/ml) ofNaCl in Formulation | Buffer, Present or Absent | pH | Deamidated Product (N16 → iso-D16) | | | Deamidated Product (N16 → D16) | | |
|---|---|---|---|---|---|---|---|---|---|
| | | | | At Beginning | First mo. | Third mo. | At Beginning | First mo. | Third mo. |
| Formulation 7 | None | Absent | 4.6 | 0.03 | 0.56 | 1.68 | 0 | 0.14 | 0.57 |
| Formulation 10 | 2.75 | Absent | 4.6 | 0.03 | 0.32 | 1.05 | 0 | 0.08 | 0.37 |
| Formulation 1 | 5.5 | Absent | 4.6 | 0.03 | 0.3 | 0.92 | 0 | 0.07 | 0.34 |
| Formulation 11 | 11 | Absent | 4.6 | 0.03 | 0.25 | 0.82 | 0 | 0.06 | 0.29 |

### [Table 11-3]

**Table 11 (Continued)**

| Formulation | Conc. (mg/ml) of NaCl in Formulation | Buffer, Present or Absent | pH | Asp Isomerized Product (D30 → iso-D30) | | |
|---|---|---|---|---|---|---|
| | | | | At Beginning | First mo. | Third mo. |
| Formulation 7 | None | Absent | 4.6 | 0.07 | 0.29 | 1.33 |
| Formulation 10 | 2.75 | Absent | 4.6 | 0.07 | 0.22 | 1.3 |
| Formulation 1 | 5.5 | Absent | 4.6 | 0.08 | 0.23 | 1.33 |
| Formulation 11 | 11 | Absent | 4.6 | 0.07 | 0.2 | 1.34 |

### [Table 12-1]

**Table 12**

| Formulation | Conc. (mg/ml) of NaCl in Formulation | Buffer, Present or Absent | pH | Deamidated Product | | | Asp Isomerized Product | | |
|---|---|---|---|---|---|---|---|---|---|
| | | | | At Beginning | First mo. | Third mo. | At Beginning | First mo. | Third mo. |
| Formulation 16 | None | Present | 4.6 | 0.09 | 0.92 | 2.85 | 0.08 | 0.55 | 2.12 |
| Formulation 5 | 2.75 | Present | 4.6 | 0.03 | 0.41 | 1.4 | 0.07 | 0.25 | 1.47 |
| Formulation 3 | 5.5 | Present | 4.6 | 0.03 | 0.36 | 1.17 | 0.07 | 0.24 | 1.41 |
| Formulation 6 | 11 | Present | 4.6 | 0.03 | 0.31 | 0.97 | 0.07 | 0.22 | 1.32 |

### [Table 12-2]

**Table 12 (Continued)**

| Formulation | Conc. (mg/ml) of NaCl in Formulation | Buffer, Present or Absent | pH | Deamidated Product (N16 → iso-D16) | | | Deamidated Product (N16 → D16) | | |
|---|---|---|---|---|---|---|---|---|---|
| | | | | At Beginning | First mo. | Third mo. | At Beginning | First mo. | Third mo. |
| Formulation 16 | None | Present | 4.6 | 0.05 | 0.55 | 1.61 | 0 | 0.18 | 0.55 |
| Formulation 5 | 2.75 | Present | 4.6 | 0.03 | 0.29 | 0.82 | 0 | 0.07 | 0.30 |
| Formulation 3 | 5.5 | Present | 4.6 | 0.03 | 0.25 | 0.67 | 0 | 0.06 | 0.26 |
| Formulation 6 | 11 | Present | 4.6 | 0.03 | 0.22 | 0.6 | 0 | 0.05 | 0.22 |

### [Table 12-3]

**Table 12 (Continued)**

| Formulation | Cone. (mg/ml) of NaCl in Formulation | Buffer, Present or Absent | pH | Asp Isomerized Product (D30 → iso-D30) | | |
|---|---|---|---|---|---|---|
| | | | | At Beginning | First mo. | Third mo. |
| Formulation 16 | None | Present | 4.6 | 0.04 | 0.36 | 1.43 |
| Formulation 5 | 2.75 | Present | 4.6 | 0.07 | 02 | 1.19 |
| Formulation 3 | 5.5 | Present | 4.6 | 0.07 | 0.19 | 1.17 |
| Formulation 6 | 11 | Present | 4.6 | 0.07 | 0.18 | 1.17 |

### [Table 13-1]

**Table 13**

| Formulation | Conc. (mg/ml) ofNaCl in Formulation | Buffer, Present Absent | pH | Deamidated Product | | | Asp Isomerized Product | | |
|---|---|---|---|---|---|---|---|---|---|
| | | | | At Beginning | First mo. | Third mo. | At Beginning | First mo. | Third mo. |
| Formulation 14 | None | Present | 4.1 | 0.07 | 0.45 | 1.64 | 0.07 | 0.26 | 1.61 |
| Formulation 16 | None | Present | 4.6 | 0.09 | 0.92 | 2.85 | 0.08 | 0.55 | 2.12 |
| Formulation 17 | None | Present | 5 | 0.09 | 1.66 | 5.22 | 0.07 | 0.79 | 2.66 |
| Formulation 2 | 5.5 | Present | 4.1 | 0.03 | 0.25 | 0.54 | 0.08 | 0.16 | 0.77 |
| Formulation 3 | 5.5 | Present | 4.6 | 0.03 | 0.36 | 1.17 | 0.07 | 0.24 | 1.41 |
| Formulation 4 | 5.5 | Present | 5 | 0.03 | 0.66 | 2.29 | 0.07 | 0.36 | 1.89 |
| Formulation 8 | 5.5 | Absent | 4.1 | 0.03 | 0.23 | 0.66 | 0.07 | 0.13 | 0.94 |
| Formulation 1 | 5.5 | Absent | 4.6 | 0.03 | 0.41 | 1.6 | 0.08 | 0.27 | 1.67 |
| Formulation 9 | 5.5 | Absent | 5 | 0.03 | 0.7 | 1.93 | 0.08 | 0.35 | 2.06 |

### [Table 13-2]

**Table 13 (Continued)**

| Formulation | Conc. (mg/ml) of NaCl in Formulation | Buffer, Present or Absent | pH | Deamidated Product (N16 → iso-D16) | | | Deamidated Product (N16 → D16) | | |
|---|---|---|---|---|---|---|---|---|---|
| | | | | At Beginning | First mo. | Third mo. | At Beginning | First mo. | Third mo. |
| Formulation 14 | None | Present | 4.1 | 0.04 | 0.29 | 0.91 | 0 | 0.11 | 0.34 |
| Formulation 16 | None | Present | 4.6 | 0.05 | 0.55 | 1.61 | 0 | 0.18 | 0.55 |
| Formulation 17 | None | Present | 5 | 0.06 | 1.02 | 3.14 | 0 | 0.31 | 0.93 |
| Formulation 2 | 5.5 | Present | 4.1 | 0.03 | 0.13 | 0.35 | 0 | 0.04 | 0.15 |
| Formulation 3 | 5.5 | Present | 4.6 | 0.03 | 0.25 | 0.67 | 0 | 0.06 | 0.26 |
| Formulation 4 | 5.5 | Present | 5 | 0.03 | 0.49 | 1.35 | 0 | 0.12 | 0.46 |
| Formulation 8 | 5.5 | Absent | 4.1 | 0.03 | 0.15 | 0.43 | 0 | 0.04 | 0.17 |
| Formulation 1 | 5.5 | Absent | 4.6 | 0.03 | 0.3 | 0.92 | 0 | 0.07 | 0.34 |
| Formulation 9 | 5.5 | Absent | 5 | 0.03 | 0.55 | 0.84 | 0 | 0.13 | 0.53 |

### [Table 13-3]

**Table 13 (Continued)**

| Formulation | Conc. (mg/ml) of NaCl in Formulation | Buffer, Present or Absent | pH | Asp Isomerized Product (D30 → iso-D30) | | |
|---|---|---|---|---|---|---|
| | | | | At Beginning | First mo. | Third mo. |
| Formulation 14 | None | Present | 4.1 | 0.04 | 0.21 | 122 |
| Formulation 16 | None | Present | 4.6 | 0.04 | 0.36 | 1.43 |
| Formulation 17 | None | Present | 5 | 0.04 | 0.46 | 1.51 |
| Formulation 2 | 5.5 | Present | 4.1 | 0.08 | 0.08 | 0.73 |
| Formulation 3 | 5.5 | Present | 4.6 | 0.07 | 0.19 | 1.17 |
| Formulation 4 | 5.5 | Present | 5 | 0.07 | 0.31 | 1.41 |
| Formulation 8 | 5.5 | Absent | 4.1 | 0.07 | 0.09 | 0.88 |
| Formulation 1 | 5.5 | Absent | 4.6 | 0.08 | 0.23 | 1.33 |
| Formulation 9 | 5.5 | Absent | 5 | 0.08 | 0.33 | 1.50 |

### [Table 14-1]

**Table 14**

| Formulation | pH | Deamidating Additive | Conc. (mg/ml) | Deamidated Product | | | Asp Isomerized Product | | |
|---|---|---|---|---|---|---|---|---|---|
| | | | | At Begin-rung | First mo. | Third mo. | At Beginning | First mo. | Third mo. |
| Formulation 1 | 4.6 | Sodium chloride | 5.5 | 0.03 | 0.41 | 1.6 | 0.08 | 0.27 | 1.67 |
| Formulation 18 | 4.6 | Calcium chloride | 5.5 | 0.03 | 0.41 | 1.62 | 0.08 | 0.25 | 1.63 |
| Formulation 19 | 4.6 | Magnesium chloride hydrate | 5.5 | 0.03 | 0.44 | 1.92 | 0.07 | 0.26 | 1.70 |
| Formulation 20 | 4.6 | Sodium bromide | 5.5 | 0.03 | 0.41 | 1.7 | 0.07 | 0.25 | 1.61 |
| Formulation 21 | 4.6 | Sodium carbonate | 5.5 | 0.03 | 0.43 | 1.79 | 0.07 | 0.25 | 1.77 |
| Formulation 22 | 4.6 | Sodium acetate hydrate | 5.5 | 0.03 | 0.44 | 1.55 | 0.07 | 0.23 | 1.57 |
| Formulation 23 | 4.6 | Trisodium citrate hydrate | 5.5 | 0.03 | 0.36 | 1.1 | 0.07 | 0.2 | 1.42 |
| Formulation 9 | 5 | Sodium chloride | 5.5 | 0.03 | 0.7 | 1.93 | 0.08 | 0.35 | 2.06 |
| Formulation 24 | 5 | Calcium chloride | 5.5 | 0.03 | 0.72 | 2.95 | 0.08 | 0.34 | 2.01 |
| Formulation 25 | 5 | Magnesium chloride hydrate | 5.5 | 0.03 | 0.76 | 322 | 0.07 | 0.34 | 2.06 |
| Formulation 26 | 5 | Sodium bromide | 5.5 | 0.03 | 0.79 | 3.4 | 0.07 | 0.35 | 2.14 |
| Formulation 27 | 5 | Sodium carbonate | 5.5 | 0.03 | 0.71 | 3.03 | 0.06 | 0.34 | 2.12 |
| Formulation 28 | 5 | Sodium acetate hydrate | 5.5 | 0.03 | 0.81 | 3.01 | 0.08 | 0.34 | 2.00 |
| Formulation 29 | 5 | Trisodium citrate hydrate | 5.5 | 0.03 | 0.6 | 2.08 | 0.07 | 0.33 | 1.91 |

### [Table 14-2]

**Table 14 (Continued)**

| Formulation | pH | Deamidating Additive | Conc. (mg/ml) | Deamidated Product (N16→iso-D16) | | | Deamidated Product (N16 → D16) | | |
|---|---|---|---|---|---|---|---|---|---|
| | | | | At Beginning | First mo. | Third mo. | At Beginning | First mo. | Third mo. |
| Formulation 1 | 4.6 | Sodium chloride | 5.5 | 0.03 | 0.3 | 0.92 | 0 | 0.07 | 0.34 |
| Formulation 18 | 4.6 | Calcium chloride | 5.5 | 0.03 | 0.31 | 0.96 | 0 | 0.08 | 0.34 |
| Formulation 19 | 4.6 | Magnesium chloride hydrate | 5.5 | 0.03 | 0.33 | 1.14 | 0 | 0.08 | 0.39 |
| Formulation 20 | 4.6 | Sodium bromide | 5.5 | 0.03 | 0.31 | 1.08 | 0 | 0.07 | 0.35 |
| Formulation 21 | 4.6 | Sodium carbonate | 5.5 | 0.03 | 0.33 | 1.04 | 0 | 0.08 | 0.38 |
| Formulation 22 | 4.6 | Sodium acetate hydrate | 5.5 | 0.03 | 0.34 | 0.89 | 0 | 0.09 | 0.36 |
| Formulation 23 | 4.6 | Trisodium citrate hydrate | 5.5 | 0.03 | 0.28 | 0.68 | 0 | 0.08 | 0.25 |
| Formulation 9 | 5 | Sodium chloride | 5.5 | 0.03 | 0.55 | 0.84 | 0 | 0.13 | 0.53 |
| Formulation 24 | 5 | Calcium chloride | 5.5 | 0.03 | 0.58 | 1.8 | 0 | 0.14 | 0.58 |
| Formulation 25 | 5 | Magnesium chloride hydrate | 5.5 | 0.03 | 0.61 | 1.99 | 0 | 0.15 | 0.62 |
| Formulation 26 | 5 | Sodium bromide | 5.5 | 0.03 | 0.64 | 2.11 | 0 | 0.15 | 0.63 |
| Formulation 27 | 5 | Sodium carbonate | 5.5 | 0.03 | 0.58 | 1.84 | 0 | 0.13 | 0.57 |
| Formulation 28 | 5 | Sodium acetate hydrate | 5.5 | 0.03 | 0.65 | 1.86 | 0 | 0.16 | 0.59 |
| Formulation 29 | 5 | Trisodium citrate hydrate | 5.5 | 0.03 | 0.48 | 121 | 0 | 0.12 | 0.44 |

### [Table 14-3]

**Table 14 (Continued)**

| Formulation | pH | Deamidating Additive | Conc. (mg/ml) | Asp Isomerized Product (D30 → iso-D30) | | |
|---|---|---|---|---|---|---|
| | | | | At Begin ning | First mo. | Third mo. |
| Formulation 1 | 4.6 | Sodium chloride | 5.5 | 0.08 | 0.23 | 1.33 |
| Formulation 18 | 4.6 | Calcium chloride | 5.5 | 0.08 | 023 | 1.31 |
| Formulation 19 | 4.6 | Magnesium chloride hydrate | 5.5 | 0.07 | 0.23 | 1.31 |
| Formulation 20 | 4.6 | Sodium bromide | 5.5 | 0.07 | 0.22 | 1.34 |
| Formulation 21 | 4.6 | Sodium carbonate | 5.5 | 0.07 | 0.23 | 1.4 |
| Formulation 22 | 4.6 | Sodium acetate hydrate | 5.5 | 0.07 | 022 | 1.27 |
| Formulation 23 | 4.6 | Trisodium citrate hydrate | 5.5 | 0.07 | 0.2 | 1.25 |
| Formulation 9 | 5 | Sodium chloride | 5.5 | 0.08 | 0.33 | 1.5 |
| Formulation 24 | 5 | Calcium chloride | 5.5 | 0.08 | 0.34 | 1.44 |
| Formulation 25 | 5 | Magnesium chloride hydrate | 5.5 | 0.07 | 0.34 | 1.45 |
| Formulation 26 | 5 | Sodium bromide | 5.5 | 0.07 | 0.35 | 1.48 |
| Formulation 27 | 5 | Sodium carbonate | 5.5 | 0.06 | 0.34 | 1.5 |
| Formulation 28 | 5 | Sodium acetate hydrate | 5.5 | 0.08 | 0.34 | 1.44 |
| Formulation 29 | 5 | Trisodium citrate hydrate | 5.5 | 0.07 | 0.33 | 1.48 |

### [Table 14-4]

**Table 14 (Continued)**

| Formulation | Conc. (mg/ml) of NaCl Formulation | Buffer, Present or Absent | pH of Formulation | Deamidated | | | Asp Isomerized | | |
|---|---|---|---|---|---|---|---|---|---|
| | | | | At Beginning | First mo. | Third mo. | At Beginning | First mo. | Third mo. |
| Formulation 12 | None | Present | 3.6 | 0.08 | 0.36 | 1.05 | 0.08 | 0.31 | 1.15 |
| Formulation 13 | None | Present | 3.8 | 0.08 | 0.35 | 125 | 0.08 | 028 | 1.34 |
| Formulation 14 | None | Present | 4.1 | 0.07 | 0.45 | 1.64 | 0.07 | 026 | 1.61 |
| Formulation 15 | None | Present | 4.4 | 0.08 | 0.66 | 2.18 | 0.07 | 0.43 | 1.92 |
| Formulation 16 | None | Present | 4.6 | 0.09 | 0.92 | 2.85 | 0.08 | 0.55 | 2.12 |
| Formulation 17 | None | Present | 5 | 0.09 | 1.66 | 522 | 0.07 | 0.79 | 2.66 |

### Example 3 (Test on Effects of Methionine):

### (1) Test Method:

A test on effects of methionine was carried out using each of Formulation 30 to 32 Preparations prepared in "Preparation of Liquid Pharmaceutical Preparations Subjected to A Test on Effects of Methionine" mentioned above.

Specifically, each formulation preparation was stored in a stability tester at 5°C, samples were then taken at sixth month, and the stability was measured by high-performance liquid chromatography.

### (2) Test Results:

The test results are shown in Tables 15 and 16.

The expression "Amount of Analogs" in the table shows a proportion (%) in a case where a total amount of teriparatide and analogs thereof contained in the formulation is defined as 100. Here, the expression "Based on Content at Beginning" in the table shows a proportion (%) of the amount of teriparatide remaining at sixth month in a case where the amount of teriparatide before storage is defined as 100.

The expression "8-Oxidized Product" in the table means a teriparatide oxidized product in which a methionine residue at the position 8 from an N-terminal of teriparatide is sulfoxidized, and the expression "18-Oxidized Product" in the table means a teriparatide oxidized product in which a methionine residue at the position 18 from an N-terminal of teriparatide is sulfoxidized, respectively.

### [Table 15]

**Table 15**

| Formulation | Based on Content at Beginning | Total Amount of Analogs | Amount of Main Analogs | |
|---|---|---|---|---|
| | | | Amount of 8-Oxidized Product | Amount of 18-Oxidized Product |
| Formulation 30 | 91.7 | 7.4 | 1.2 | 1.9 |
| Formulation 31 | 94.6 | 4.6 | 0.6 | 1.0 |
| Formulation 32 | 91.7 | 9.8 | 3.5 | 4.0 |

### [Table 16]

**Table 16**

| Formulation | L-Methionine Concentration (µg/g) | Ratio of L-Methionine: Teriparatide | Buffer, Presence or Absence | pH |
|---|---|---|---|---|
| Formulation 30 | 35 | 35:141 | Absent | 5 |
| Formulation 31 | 100 | 100:141 | Absent | 5 |
| Formulation 32 | None | 0:141 | Absent | 4.1 |

### Example 4 (Stability Test Relating to Filled Containers):

### (1) Test Method:

A stability test was carried out using each of Formulation 33 to 40 Ampule Preparations prepared in "Liquid Pharmaceutical Preparations Subjected to Stability Test Relating to Filled Containers" mentioned above and Formulation 33 to 40 Syringe Preparations prepared in "Liquid Pharmaceutical Preparations Subjected to Stability Test Relating to Filled Containers" mentioned above.

Specifically, each formulation preparation was stored in a stability tester at 25°C / 60% RH, and samples were taken at third month. The stability was measured by high-performance liquid chromatography.

### (2) Test Results:

The test results are shown in Tables 17 and 18.

The expression "Based on Content at Beginning" in the table shows a proportion (%) of the amount of teriparatide remaining at third month in a case where the amount of teriparatide before storage is defined as 100. The expression "Total Amount of Analogs" in the table shows a proportion (%) of a total amount of analogs present at third month in a case where the amount of teriparatide and a total amount of analogs present at third month is defined as 100.

### [Table 17]

**Table 17: Stability of Glass Ampule Preparations**

| Formulation | Based on Content at Beginning | Total Amount of Analogs |
|---|---|---|
| Formulation 33 | 94.0 | 72 |
| Formulation 34 | 92.2 | 7.4 |
| Formulation 35 | 93.7 | 7.0 |
| Formulation 36 | 94.0 | 7.0 |
| Formulation 37 | 93.3 | 7.0 |
| Formulation 38 | 93.7 | 7.0 |
| Formulation 39 | 93.3 | 6.7 |
| Formulation 40 | 93.9 | 7.1 |

### [Table 18]

**Table 18: Stability of Plastic Syringe Preparations**

| Formulation | Based on Content at Beginning | Total Amount of Analogs |
|---|---|---|
| Formulation 33 | 92.1 | 8.0 |
| Formulation 34 | 90.0 | 8.3 |
| Formulation 35 | 90.4 | 10.5 |
| Formulation 36 | 92.1 | 7.4 |
| Formulation 37 | 91.8 | 7.5 |
| Formulation 38 | 90.6 | 8.0 |
| Formulation 39 | 87.3 | 7.3 |
| Formulation 40 | 90.0 | 8.1 |

### Example 5 (Stability Test Relating to Control Comparisons):

### (1) Test Method:

A stability test was carried out using each of Formulation 41 and 42 Preparations prepared in "Liquid Pharmaceutical Preparations Subjected to Stability Test Relating to Control Comparisons" mentioned above and Formulation 38 Syringe Preparation prepared in "Liquid Pharmaceutical Preparations Subjected to Stability Test Relating to Filled Containers" mentioned above.

Specifically, each formulation was stored in a stability tester at 25°C / 60% RH, and samples were taken at first month and at third month. The stability was measured by high-performance liquid chromatography.

### (2) Test Results:

The test results are shown in Table 19.

The expression "Based on Content at Beginning (After Three-Mo. Storage)" in the table shows a proportion (%) of the amount of teriparatide remaining at third month in a case where the amount of teriparatide before storage is defined as 100. The expression "Total Amount of Analogs (After Three-Mo. Storage)" in the table shows a proportion (%) of a total amount of analogs present at third month in a case where the amount of teriparatide and a total amount of analogs present at third month is defined as 100.

### [Table 19]

**Table 19: Stability of Plastic Syringe Preparations**

| Formulation | pH | Buffer, Presence or Absence | Based on Content at Beginning (After Three-Mo. Storage) | Total Amount of Analogs (After Three-Mo. Storage) |
|---|---|---|---|---|
| Formulation 41 | 4.1 | Present | 83.1 | 13.7 |
| Formulation 42 | 4.1 | Present | 90.1 | 9.0 |
| Formulation 38 | 4.6 | Absent | 90.6 | 8.0 |

### Example 6 (Stability Test Relating to Inhibition of Specified Analogs):

### (1) Test Method:

A stability test relating to inhibition of specified analogs was carried out using each of Formulation 33 and 38 Preparations prepared in "Preparation of Liquid Pharmaceutical Preparations Subjected to Stability Test Relating to Filled Containers" mentioned above and Formulation 62 Preparation prepared in "Preparation of Control Liquid Pharmaceutical Preparation" mentioned above.

Specifically, each formulation preparation was stored in a stability tester at 5°C, and samples were taken in the time course. The stability was measured by high-performance liquid chromatography. The conditions for the high-performance liquid chromatography are as follows.

(1) Detector: ultraviolet absorptiometer (measurement wavelength: 214 nm);
(2) Column: a stainless steel tube having an inner diameter of 4.6 mm and a length of 150 mm, packed with 3.5 µm octadecylsilylated silica gel for liquid chromatography (Zorbax 300SB-C18 manufactured by Agilent Technologies or an equivalent product);
(3) Column temperature: a constant temperature around 40°C;
(4) Mobile phases
Mobile phase A: The amount 28.4 g of anhydrous sodium sulfate is dissolved in 900 mL of water, phosphoric acid is added thereto to adjust its pH to 2.3, and water is then added to make up a volume of 1000 mL. One-hundred milliliters of acetonitrile is added to 900 mL of this solution.
Mobile phase B: The amount 28.4 g of anhydrous sodium sulfate is dissolved in 900 mL of water, phosphoric acid is added thereto to adjust its pH to 2.3, and water is then added to make a volume of 1000 mL. Five-hundred milliliters of acetonitrile is added to 500 mL of this solution.
(5) Feeding liquids of mobile phases
The concentration gradient is controlled by changing a mixing ratio of the mobile phase A and the mobile phase B as shown in the following Table 20.
[Table 20]

**Table 20: Controlling Concentration Gradients**

| Time (minute) After Sample Injection | Mobile Phase A (vol %) | Mobile Phase B (vol %) |
|---|---|---|
| 0 to 5 | 100 → 65 | 0 → 35 |
| 5 to 35 | 65 → 60 | 35 → 40 |
| 35 to 45 | 60 → 0 | 40 → 100 |

(6) Flow rate: 1.0 mL per minute;
(7) Detection time: 45 minutes after injection of a sample solution. However, the detection is made from the tail end of the solvent peak.

### (2) Test Results:

The amounts of analogs at a relative retention time of 0.72 of each formulation are shown in Table 21.

The expression "Content at Beginning" in the table shows a proportion (%) of the amount of analogs at a relative retention time of 0.72 in a case where the amount of teriparatide and a total amount of analogs before storage is defined as 100. The expression "Content at Third Month" in the table shows a proportion (%) of the amount of analogs at a relative retention time of 0.72 at third month in a case where the amount of teriparatide and a total amount of analogs at third month is defined as 100.

### [Table 21]

**Table 21: Amount of Analogs at Relative Retention Time of 0.72**

| Formulation | Content at Beginning | Content at Third Month |
|---|---|---|
| Formulation 33 | 0.07 | 0.27 |
| Formulation 38 | 0.00 | 0.02 |
| Formulation 62 | 0.03 | 0.03 |

The structure of the analogs at a relative retention time of 0.72 is not clarified. However, since the conditions for high-performance liquid chromatography (1) to (7) mentioned above are substantially identical to the conditions described in [0087] to [0089] of the Patent Publication 5, the inventors consider that it is highly possible that the analogs are any one of analogs 9' to 11' described in Patent Publication 5 (relative retention time being from 0.69 to 0.74; see, Table 3 of Patent Publication 5) or a mixture thereof.

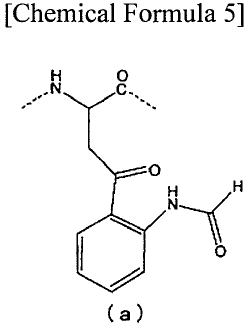

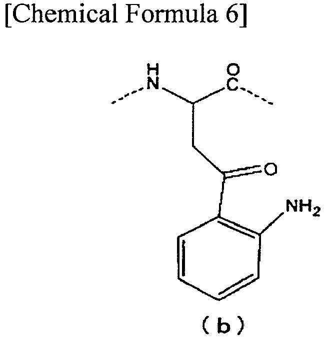

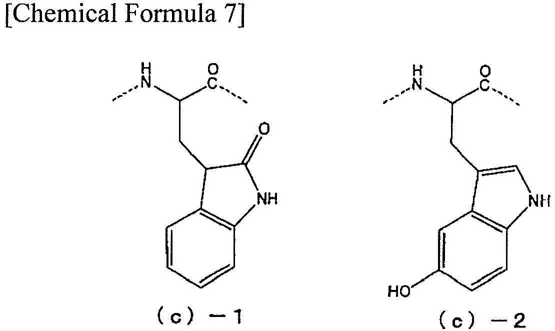

Here, the analog 9' described in Patent Publication 5 means a teriparatide analog (human PTH(1-34)-Trp23 [dioxide]) in which a residue corresponding to tryptophan at the position 23 of teriparatide is the above (a) residue, and the other structures are identical to the original teriparatide. The analog 10' means a teriparatide analog (human PTH(1-34)-Trp23 [monoxide]) in which a residue corresponding to tryptophan at the position 23 of teriparatide is a residue represented by the above (c)-1 or the above (c)-2, and the other structures are identical to the original teriparatide. The analog 11' means a teriparatide analog (human PTH(1-34)-Trp23[dioxide-formate detachment]) in which a residue corresponding to tryptophan at the position 23 of teriparatide is the above (b) residue, and the other structures are identical to the original teriparatide.

Example 7 (Stability Test Relating to Evaluation of Additives):

### (1) Test Method:

A stability test relating to the evaluation of additives was carried out using Formulations 43 to 61 prepared in "Preparation of Liquid Pharmaceutical Preparations Subjected to Stability Test Relating to Evaluation of Additives" mentioned above.

Specifically, each formulation preparation was stored in a stability tester at 25°C / 60% RH, and samples were then taken at first month and at third month. The stability was measured by high-performance liquid chromatography.

### (2) Test Results:

The test results are shown in Table 22.

"-" in the table means the fact that the measurement was not taken.

The expression "Content at Beginning" in the table shows a proportion (%) of a total amount of analogs present in a case where the amount of teriparatide and a total amount of analogs before storage is defined as 100. The expression "Content at First Month" in the table shows a proportion (%) of a total amount of analogs present at first month in a case where the amount of teriparatide and a total amount of analogs present at first month is defined as 100. The expression "Content at Third Month" in the table shows a proportion (%) of a total amount of analogs present at third month in a case where the amount of teriparatide and a total amount of analogs present at third month is defined as 100.

### [Table 22]

**Table 22**

| Formulation | Additives | Total Amount of Analogs | | |
|---|---|---|---|---|
| | | Content at Beginning | Content at First Month | Content at Third Month |
| Formulation 43 | None | 0.80 | 4.68 | 9.59 |
| Formulation 44 | D-Mannitol | 0.94 | 3.85 | 8.71 |
| Formulation 45 | D-Mannitol | 0.96 | 5.96 | 1527 |
| Formulation 46 | L-Proline | 0.97 | 4.59 | 1026 |
| Formulation 47 | Betaine | 127 | 4.70 | 10.94 |
| Formulation 48 | Ectoine | 1.49 | 6.09 | 13.86 |
| Formulation 49 | Hydroxyectoine | 130 | 4.95 | 1235 |
| Formulation 50 | L-Arginine hydrochloride | 0.81 | 4.63 | 11.09 |
| Formulation 51 | L-Histidine | 1.43 | 723 | 16.98 |
| Formulation 52 | 2-Hydroxypropyl-β-cyclodextrin | 0.96 | 8.04 | 1721 |
| Formulation 53 | γ-Cyclodextrin | 1.30 | 1022 | 26.07 |
| Formulation 54 | α-Cyclodextrin | 0.85 | 4.58 | 7.57 |
| Formulation 55 | β-Cyclodextrin | 0.85 | 326 | 8.12 |
| Formulation 56 | L-Lysine hydrochloride | 133 | 4.30 | 9.06 |
| Formulation 57 | N-Acetyl-DL-tryptophan | 3.41 | 7.61 | 17.17 |
| Formulation 58 | N-Acetyl-DL-tryptophan | 1.74 | 5.09 | 12.48 |
| Formulation 60 | None | 1.06 | 24.44 | - |
| Formulation 61 | None | 1.00 | 5.42 | 17.53 |
| Formulation 59 | N-Acetylarginine | 1.33 | 3.81 | 7.80 |

### Example 8 (Photostability Test):

### (1) Test Method:

Formulation 38 Syringe Preparation prepared in "Liquid Pharmaceutical Preparations Subjected to Stability Test Relating to Filled Containers" mentioned above or the same preparation packaged with paper box or the like was loaded to a commercially available photostability testing apparatus, with a D65 lamp as a light source (illuminance: 2500 lx), under conditions of 5°C ± 3°C and exposed for a period satisfying 1200000 lx•hr or more and 200 w•h/m² over a period of 20 days, or a period of satisfying 600000 lx•hr or more over a period of 10 days. After the exposure, the samples were taken, and subjected to high-performance liquid chromatography to measure a total amount of analogs, the 8-oxidized product and the 18-oxidized product in the preparation. Here, the same preparation packaged with paper box or the like refers to a syringe preparation incorporated into a device, the preparation-incorporated device being further blister packaged, and then included in an individually packaged box.

### (2) Test Results:

The measurement results are shown in the following table. Each numerical figure in the columns of the measurement timing shows a proportion (%) of the amount of the corresponding oxidized product when the amount of teriparatide and a total amount of analogs before storage are defined as 100.

### [Table 23]

**Table 23**

| Measurement Item | Number of Repeats | Measurement Timing | | | | |
|---|---|---|---|---|---|---|
| | | At Beginning | 600000 lx•hr | | 1200000 lx•hr | |
| | | | Exposed in Syringe State | Exposed in Final Packaged State (Paper Box) | Exposed in Syringe State | Exposed in Final Packaged State (Paper Box) |
| Purity Test (Analogs) | | | | | | |
| Met (O)⁸ Product | 1 | 0.2 | 1.4 | 0.2 | 2.3 | 0.2 |
| | 2 | 0.2 | 1.4 | 0.2 | 2.5 | 0.2 |
| | 3 | 02 | 1.4 | 0.2 | 2.7 | 0.2 |
| | Mean | 0.2 | 1.4 | 0.2 | 2.5 | 0.2 |
| Met (O)¹⁸ Product | 1 | 0.2 | 2.0 | 0.2 | 3.6 | 0.2 |
| | 2 | 02 | 2.0 | 0.2 | 3.8 | 0.2 |
| | 3 | 02 | 2.0 | 0.2 | 4.1 | 0.2 |
| | Mean | 02 | 2.0 | 0.2 | 3.8 | 0.2 |
| Total Amount | 1 | 1.8 | 63 | 1.9 | 10.5 | 1.9 |
| | 2 | 1.8 | 6.3 | 1.9 | 10.9 | 1.9 |
| | 3 | 1.8 | 6.2 | 2.0 | 11.6 | 2.0 |
| | Mean | 1.8 | 6.3 | 1.9 | 11.0 | 1.9 |

### INDUSTRIAL APPLICABILITY

The liquid pharmaceutical preparation of the present invention is excellent from the viewpoint of physical properties and pharmacokinetics. Each of the methods of the present invention (a method for inhibiting a deamidation reaction, a method for storage, and a method for inhibiting formation of analogs) is also an epoch-making method for controlling a main drug. The present invention is very useful in the pharmaceutical industries.

## Claims

1. A liquid pharmaceutical preparation comprising Component 1 and Component 2:
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.

2. The liquid pharmaceutical preparation according to claim 1,
wherein a pH is less than 5.0.

3. A liquid pharmaceutical preparation comprising Component 1 and Component 2, provided that a pH is less than 5.0:
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation substantially comprising a buffer, provided that when Component 1 is a teriparatide salt, a salt detached from Component 1 does not fall under said buffer.

4. A liquid pharmaceutical preparation comprising Component 1 and Component 2, provided that a pH is less than 5.0:
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation not substantially comprising a buffer, provided that when Component 1 is a teriparatide salt, a salt detached from Component 1 does not fall under said buffer.

5. A liquid pharmaceutical preparation comprising Component 1 and Component 2, provided that a pH is 5.0 or more:
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, the liquid pharmaceutical preparation not substantially comprising a buffer, provided that when Component 1 is a teriparatide salt, a salt detached from Component 1 does not fall under said buffer.

6. The liquid pharmaceutical preparation according to any one of the claims 1 to 5, wherein the mass ratio of Component 1 to Component 2 is 1 : 20 or 20 or more.

7. A liquid pharmaceutical preparation comprising Component 1:
wherein Component 1 is teriparatide or a salt thereof, and
wherein a pH is from 3.6 to 4.1.

8. A liquid pharmaceutical preparation comprising Component 1 and Component 3:
wherein Component 1 is teriparatide or a salt thereof; and
wherein Component 3 is methionine,
wherein the mass ratio of Component 1 to Component 3 is from 1 : 0.2 to 1.0.

9. A liquid pharmaceutical preparation comprising Component 1 and Component 4:
wherein Component 1 is teriparatide or a salt thereof; and
wherein Component 4 is D-mannitol.

10. A liquid pharmaceutical preparation comprising Component 1,
wherein Component 1 is teriparatide or a salt thereof,
the liquid pharmaceutical preparation being filled in a glass container for medical use, the liquid pharmaceutical preparation not substantially comprising a buffer, and having a pH of exceeding 4.0 and 5.0 or less, provided that when Component 1 is a teriparatide salt, a salt detached from Component 1 does not fall under said buffer.

11. A liquid pharmaceutical preparation comprising Component 1:
wherein Component 1 is teriparatide or a salt thereof,
the liquid pharmaceutical preparation being filled in a plastic container for medical use, and having a pH of from 4.0 to 4.2, the liquid pharmaceutical preparation substantially comprising a buffer, provided that when Component 1 is a teriparatide salt, a salt detached from Component 1 does not fall under said buffer, and the liquid pharmaceutical preparation further comprising Component 4:
wherein Component 4 is D-mannitol.

12. A liquid pharmaceutical preparation comprising Component 1 and Component 5:
wherein Component 1 is teriparatide or a salt thereof; and
wherein Component 5 is one or more components selected from the group consisting of L-proline, betaine, ectoine, hydroxyectoine, L-arginine hydrochloride, L-histidine, 2-hydroxypropyl-β-cyclodextrin, α-cyclodextrin, β-cyclodextrin, γ-cyclodextrin, N-acetyl-arginine, L-lysine hydrochloride, and N-acetyl-DL-tryptophan.

13. The liquid pharmaceutical preparation according to claim 12,
wherein Component 5 is one or more components selected from the group consisting of α-cyclodextrin, β-cyclodextrin, N-acetyl-arginine, L-proline, L-arginine hydrochloride, and L-lysine hydrochloride.

14. The liquid pharmaceutical preparation according to any one of claims 1 to 6, wherein the Component 2 is one or more salts selected from sodium chloride, calcium chloride, magnesium chloride, sodium bromide, sodium acetate, trisodium citrate, and sodium carbonate.

15. The liquid pharmaceutical preparation according to claim 14,
wherein the Component 2 is sodium chloride and/or trisodium citrate.

16. The liquid pharmaceutical preparation according to claim 3 or 4,
wherein a pH is 4.1 or more and 4.6 or less.

17. The liquid pharmaceutical preparation according to claim 5,
wherein a pH is 5.0.

18. The liquid pharmaceutical preparation according to any one of claims 1 to 17, wherein Component 1 is teriparatide acetate.

19. The liquid pharmaceutical preparation according to any one of claims 1 to 18, wherein the Component 1 content is from 25 to 30 µg, in terms of teriparatide.

20. The liquid pharmaceutical preparation according to any one of claims 1 to 19, for use in subcutaneous administration in human.

21. A method for inhibiting a deamidation reaction of Component 1 in a liquid pharmaceutical preparation comprising Component 1, comprising formulating the liquid pharmaceutical preparation to further include Component 2,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.

22. The method according to claim 21, wherein a pH of the liquid pharmaceutical preparation is less than 5.0.

23. A method for inhibiting a deamidation reaction of Component 1 in a liquid pharmaceutical preparation comprising Component 1 under conditions of a pH of less than 5.0, comprising formulating the liquid pharmaceutical preparation to further include Component 2, and also substantially include a buffer,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under said buffer.

24. A method for inhibiting a deamidation reaction of Component 1 in a liquid pharmaceutical preparation comprising Component 1 under conditions of a pH of less than 5.0, comprising formulating the liquid pharmaceutical preparation to further include Component 2, but not substantially include a buffer,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under said buffer.

25. A method for inhibiting a deamidation reaction of Component 1 in a liquid pharmaceutical preparation comprising Component 1 under conditions of a pH of 5.0 or more, comprising formulating the liquid pharmaceutical preparation to further include Component 2, but not substantially include a buffer,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under said buffer.

26. The method according to claim 25, wherein the inhibition of a deamidation reaction is inhibition of formation of a deamidated product of Component 1, wherein the deamidated product has a change of a residue at the position 16 from an N-terminal of Component 1 from an asparagine residue to an isoaspartic acid residue.

27. The method according to any one of claims 21 to 26, wherein the Component 2 is one or more salts selected from sodium chloride, calcium chloride, magnesium chloride, sodium bromide, sodium acetate, trisodium citrate, and sodium carbonate, provided that Component 2 is a component different from Component 1.

28. A method for inhibiting a deamidation reaction of Component 1 in a liquid pharmaceutical preparation comprising Component 1:
wherein Component 1 is teriparatide or a salt thereof,
wherein a pH of the liquid pharmaceutical preparation is adjusted to from 3.6 to 4.1.

29. A method for inhibiting isomerization of an aspartic acid residue of Component 1 in a liquid pharmaceutical preparation comprising Component 1, comprising formulating the liquid pharmaceutical preparation to further include Component 2,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1.

30. A method for inhibiting isomerization of an aspartic acid residue of Component 1 in a liquid pharmaceutical preparation comprising Component 1, comprising formulating the liquid pharmaceutical preparation to further include Component 2, and also substantially include a buffer,
wherein Component 1 is teriparatide or a salt thereof; and
wherein Component 2 is one or more salts selected from sodium salts, calcium salts, and magnesium salts, and/or one or more salts selected from hydrochlorides, hydrobromides, acetates, citrates, and carbonates, provided that Component 2 is a component different from Component 1, and provided that when Component 1 is a teriparatide salt, a salt detached from Component 1 does not fall under said buffer.

31. The method according to claim 30, wherein the inhibition of isomerization of an aspartic acid residue is inhibition of isomerization of an aspartic acid residue of Component 1, wherein the isomerization of a residue at the position 30 from an N-terminal of Component 1 is a change of from an aspartic acid residue to an isoaspartic acid residue.

32. The method according to any one of claims 29 to 31, wherein Component 2 is one or more salts selected from sodium chloride, calcium chloride, magnesium chloride, sodium bromide, sodium acetate, trisodium citrate, and sodium carbonate, provided that Component 2 is a component different from Component 1.

33. A method for inhibiting formation of an oxidized product of Component 1 in a liquid pharmaceutical preparation comprising Component 1,
wherein Component 1 is teriparatide or a salt thereof,
the method comprising formulating the liquid pharmaceutical preparation to not substantially include a buffer, provided that when Component 1 is a teriparatide salt, a salt detached from Component 1 does not fall under said buffer, and adjusting a pH of a liquid pharmaceutical preparation to exceeding 4.0 and 5.0 or less, and also formulating the liquid pharmaceutical preparation to include Component 3,
wherein Component 3 is methionine,
so as to have a mass ratio of Component 1 to Component 3 of from 1 : 0.2 to 1.0, wherein an oxidized product of Component 1 is teriparatide oxidized product in which a methionine residue at the position 8 from an N-terminal of teriparatide is sulfoxidized or a salt thereof.

34. A method for inhibiting formation of an oxidized product of Component 1 in a liquid pharmaceutical preparation comprising Component 1:
wherein Component 1 is teriparatide or a salt thereof,
the method comprising formulating the liquid pharmaceutical preparation to include Component 4,
wherein Component 4 is D-mannitol,
wherein the oxidized product of Component 1 is an oxidized product in which a tryptophan residue at the position 23 from an N-terminal of Component 1 is oxidized, and the other residues are identical to the corresponding residues of teriparatide.

35. A method for storing a liquid pharmaceutical preparation comprising Component 1,
wherein Component 1 is teriparatide or a salt thereof,
the liquid pharmaceutical preparation being filled in a glass container for medical use, the method comprising formulating the liquid pharmaceutical preparation to not substantially include a buffer, provided that when Component 1 is a teriparatide salt, a salt detached from Component 1 does not fall under said buffer, and adjusting a pH of the liquid pharmaceutical preparation to exceeding 4.0 and 5.0 or less.

36. A method for storing a liquid pharmaceutical preparation comprising Component 1,
wherein Component 1 is teriparatide or a salt thereof,
the liquid pharmaceutical preparation being filled in a plastic container for medical use, the method comprising formulating the liquid pharmaceutical preparation to substantially include a buffer, and formulating the liquid pharmaceutical preparation to further include Component 4, provided that when Component 1 is a teriparatide salt, a salt detached from Component 1 does not fall under said buffer,
wherein Component 4 is mannitol, and
adjusting a pH of the liquid pharmaceutical preparation to exceeding 4.0 and 5.0 or less.

37. A method for storing a liquid pharmaceutical preparation comprising Component 1:
wherein Component 1 is teriparatide or a salt thereof,
the method comprising formulating the liquid pharmaceutical preparation to include Component 5:
wherein Component 5 is one or more components selected from the group consisting of L-proline, betaine, ectoine, hydroxyectoine, L-arginine hydrochloride, L-histidine, 2-hydroxypropyl-β-cyclodextrin, α-cyclodextrin, β-cyclodextrin, γ-cyclodextrin, N-acetyl-arginine, L-lysine hydrochloride, and N-acetyl-DL-tryptophan.

38. The method according to claim 37, wherein Component 5 is one or more components selected from the group consisting of α-cyclodextrin, β-cyclodextrin, N-acetyl-arginine, L-proline, L-arginine hydrochloride, and L-lysine hydrochloride.

39. An analog of Component 1 wherein a residue at the position 16 from an N-terminal of Component 1 is changed from an asparagine residue to an isoaspartic acid residue, and the other residues are identical to the corresponding residues of teriparatide,
wherein Component 1 is teriparatide or a salt thereof.

40. An analog of Component 1 wherein a residue at the position 16 from an N-terminal of Component 1 is changed from an asparagine residue to an aspartic acid residue, and the other residues are identical to the corresponding residues of teriparatide,
wherein Component 1 is teriparatide or a salt thereof.

41. An analog of Component 1 wherein a residue at the position 30 from an N-terminal of Component 1 is changed from an aspartic acid residue to an isoaspartic acid residue, and the other residues are identical to the corresponding residues of teriparatide,
wherein Component 1 is teriparatide or a salt thereof.

42. An analog of Component 1 wherein a residue at the position 10 from an N-terminal of Component 1 is changed from an asparagine residue to an aspartic acid residue or an isoaspartic acid residue, and a residue at the position 30 from an N-terminal of Component 1 is changed from an aspartic acid residue to an isoaspartic acid residue, and the other residues are identical to the corresponding residues of teriparatide, wherein Component 1 is teriparatide or a salt thereof.

43. A method for testing quality of a liquid pharmaceutical preparation comprising Component 1, comprising detecting and/or quantifying an analog as defined in any one of claims 39 to 42,
wherein Component 1 is teriparatide or a salt thereof.

44. A method for inhibiting formation of an oxidized product in a liquid pharmaceutical syringe preparation comprising Component 1, comprising shading the preparation from light, wherein the oxidized product is a teriparatide oxidized product in which a methionine residue at the position 18 from an N-terminal of teriparatide is sulfoxidized, or a salt thereof,
wherein Component 1 is teriparatide or a salt thereof.
